# EUROPEAN PATENT APPLICATION

(11) **EP 1 391 211 A1**
(43) Date of publication of application: **25.02.2004**
(21) Application number: 02720611.9
(22) Date of filing: 25.04.2002
(51) Int. Cl.: A61K 45/06, A61K 31/405, A61K 31/277, A61K 31/353, A61P 19/00, A61P 19/02, A61P 19/04, A61P 29/00, C07D 209/40, C12N 5/06, C12N 5/08

(54) **CHONDROGENESIS PROMOTERS**

(30) Priority: 27.04.2001 JP 2001131252
(71) Applicant: CHUGAI SEIYAKU KABUSHIKI KAISHA, Tokyo, 115-8543 (JP)
(72) Inventor: OKAZAKI, Makoto, c/o CHUGAI SEIYAKU KABUSHIKI, Gotenba-shi, Shizuoka 412-8513 (JP)
(74) Representative: HOFFMANN EITLE
(86) International application number: PCT/JP2002/004176
(87) International publication number: WO 2002/087620

(57) **Abstract**

A chondrogenesis promoter comprising a low molecular weight compound having a chondrogenesis promoting effect and a compound with MEK1 inhibitory activity.

## Description

### Technical Field

The present invention relates to chondrogenesis promoters.

### Background Art

Cartilage in the body generally consists of chondrocytes and fibroblasts that are specialized connective tissue cells, and an amorphous gel-like matrix in which they are embedded, and it forms a part of the supportive tissue of the body.

In warm-blooded animals including humans, cartilage forms the skeleton, joints, tracheae, auricula, nose and the like. That is, it performs a central role in functions that are indispensable to the survival of warm-blooded animals, including acting as a template for bone during growth (growth cartilage), and contributing to smooth joint movement (articular cartilage), respiration (tracheal cartilage, nasal cartilage) and hearing (auricular cartilage). Thus, degeneration or destruction of these types of cartilage causes various degrees of detriment to the body depending on the site and severity of degeneration or destruction.

For example, among the aforementioned functions in which cartilage plays a role (growth, joints, respiration, hearing, etc.), the smooth movement of joints is particularly impaired by degeneration or destruction of articular cartilage in such conditions as chronic rheumatoid arthritis or osteoarthritis. Degeneration or destruction of articular cartilage is believed to be the major cause of the walking difficulty that results from such diseases.

On the other hand, The prospect of suppressing articular degeneration or destruction or of promoting chondrogenesis has been raised as a possible method of treating conditions such as chronic rheumatoid arthritis and osteoarthritis (J. Rheum. 22(1), Suppl. 43:136-139, 1995, Lab. Invest. 78(2):133-142).

Several different organism-derived substances and low molecular weight substances are known to have effects of promoting chondrogenesis or of inducing proliferation of chondrocytes. It has been reported that the chondrogenesis promoting effect was confirmed with, for example, growth factors such as TGF-β (Transforming growth factor β); BMP-2 (J. Bone Joint Surg. 79-A(10):1452-1463, 1997); concanavalin A which is a type of lectin (J. Biol. Chem., 265:10125-10131, 1990); as well as low molecular weight substances such as osteogenin (BMP-7), vitamin D derivatives (1α, 25-D₃) (Cancer Res., 49:5435-5442, 1989), vitamin A derivatives (retinoic acid) (Dev. Biol., 130:767-773, 1988), vanadates (J. Cell Biol., 104:311-319, 1987), benzamides (J. Embryol. Exp. Morphol., 85:163-175, 1985), benzyl β-D-xyloside (Biochem. J., 224:977-988, 1984), triiodothyronines (T₃) (Endocrinology, 111:462-468, 1982), prostaglandin derivatives (PGE₂, U44069) (Prostaglandin, 19:391-406, 1980), dbcAMP (J. Cell Physiol., 100:63-76, 1979), 8-Br-cAMP (J. Cell Physiol., 100:63-76, 1979), TAK-778 (Biochem. Biophys. Res. Com., 261:131-138,1999), and the like. Furthermore, WO00/44722 discloses that indolin-2-one derivatives of specific structure have the chondrogenesis promoting effect.

Of these organism-derived substances and low molecular weight substances, TGF-β holds the most promise as a useful treatment agent, and TGF-β1, which is one isoform of TGF-β, has been reported to promote chondrogenesis when intraarticularly administered (Lab. Invest. 71(2):279-290, 1994). Also, since TGF-β1 suppresses arthritis-induced defect of proteoglycans in articular cartilage in animal models with experimental arthritis, or stated differently, since it inhibits destruction of articular cartilage due to its anabolic effect on articular cartilage when administered intraarticularly, its possibility as a useful treatment agent for articular disease such as rheumatism has been suggested (Lab. Invest. 78(2):133-142, 1998).

However, even TGF-β, which holds the most promise as a useful treatment agent, has been reported to provoke synovitis even while promoting chondrogenesis, and this therefore poses a serious problem for its use as a treatment agent for aforementioned articular diseases (Lab. Invest. 71(2):279-290, 1994), for which reason it has not been applied in the clinic as a treatment agent for such conditions. In summary, then, no practical treatment agent therapy exists that is based on promoting chondrogenesis.

On the other hand, it is known that transplantation of cells removed from the living body is useful to the repair of damaged cartilage, and the transplantation has been already clinically applied. Chondrocytes (Clin. Orthop. Res. 367S: S147-S155, 1999) and mesenchymal precursor cells (Clin. Orthop. Res. 367S: S156-S162, 1999) are present for the cells used in the transplantation, and, particularly, the autologous chondrocyte transplantation was recently put into practical use and made some achievement. There arose, however, a problem that while the removed chondrocytes were cultured in vitro to proliferate, they dedifferentiated to lose the phenotype as chondrocytes, so as to result in degradation of the quality of repaired tissue. That is, it is presently difficult to repair cartilage defect with functionally perfect hyaline cartilage even by the treatment adopting the cell transplantation.

MAPK (Mitogen Activated Protein Kinases) are signaling pathways playing critical roles in proliferation and differentiation of cells, which are activated in response to a variety of extracellular stimuli, e.g., growth factors and physiochemical stress, to regulate the gene expression. Principal MAPKs include p44/42(Erk), p38, and JNK, each of which transmits specific signals. MEK1 is MAPKK (Mitogen Activated Protein Kinase Kinase) located upstream of p44/42MAPK, and plays a role of activating p44/42MAPK in response to a growth factor. The activation stated herein is phosphorylation of proteins. Even though there is no report stating that MEK1 directly controls chondrogenesis, it is reported (Nature 389: 618-622, 1997) that p44/42MAPK, downstream of MEK1, negatively controls Smad, the molecules that transmit TGF-β and BMP signals; furthermore, it is reported (Exp. Cell Res. 250: 351-363, 1999) that MEK1 inhibitors enhance the induction effect of TGF-β and BMP on differentiation of mouse cartilage precursor cell into chondrocyte. Compounds known as MEK1 inhibitors are, for example, PD98059 (J. Biol. Chem. 270: 27489-27494, 1995), U0126 (J. Biol. Chem. 273: 18623-18632, 1998), and the like. However, the MEK1 inhibitors have never independently presented satisfactory chondrogenesis promoting effect, and there are desires for agents that can demonstrate satisfactory effect.

### Disclosure of the Invention

It is an object of the present invention to provide a chondrogenesis promoter demonstrating outstanding chondrogenesis promotion effect, as compared with the agents by the aforementioned prior art.

It is another object of the invention to provide a cartilage repair agent, a chondrocyte phenotype maintaining agent, a bone fracture repair promoter, a therapeutic or prophylactic agent for a cartilage disease, a reagent for biological, physical, or chemical research on cartilage, and an agent for chondrocyte transplantation, each of which contains the foregoing chondrogenesis promoter. It is yet another object of the invention to provide a method for promoting chondrogenesis and a therapeutic or prophylactic method for a cartilage disease, using the aforementioned chondrogenesis promoter. It is yet another object of the invention to provide a low molecular weight compound being a component of the foregoing chondrogenesis promoter, a therapeutic or prophylactic method for a cartilage disease utilizing the low molecular weight compound, and a kit of the foregoing chondrogenesis promoter. It is a yet further object of the present invention to provide a method for proliferating chondrocyte and a method for producing chondrocyte, and chondrocytes obtained by such methods.

The Inventors conducted elaborate research in order to achieve the above objects and found that a combination of a low molecular weight compound having a chondrogenesis promoting effect (e.g., a low molecular weight compound containing an indolin-2-one derivative of specific structure, etc.) with a compound with MEK1 inhibitory activity, demonstrated unexpectedly dramatic, synergistic chondrogenesis promoting effect, thus completing the present invention based on the finding.

That is, the present invention provides a chondrogenesis promoter comprising a low molecular weight compound having a chondrogenesis promoting effect and a compound with MEK1 inhibitory activity.

The above-stated low molecular weight compound having a chondrogenesis promoting effect is preferably a compound represented by general formula (I) below or a salt thereof: 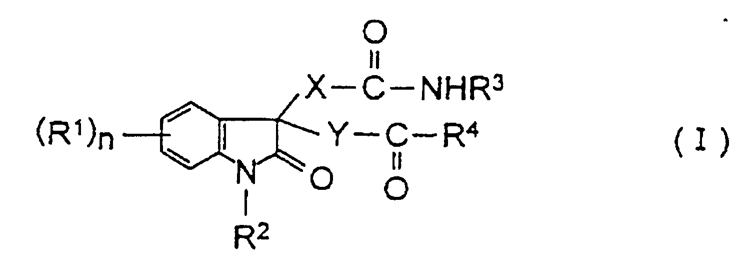 wherein
R¹ represents a halogen atom, a lower alkyl group, a lower alkoxy group, a hydroxyl group, a nitro group, a trifluoromethyl group, a lower alkylthio group, an acyl group, a carboxyl group, a mercapto group, or an amino group with an optional substituent;
R² represents a hydrogen atom, a lower alkyl group with an optional substituent, a lower alkenyl group with an optional substituent, a lower alkynyl group with an optional substituent, a lower alkoxy group with an optional substituent, an acyl group with an optional substituent, an aryl group with an optional substituent, or a heterocyclic group with an optional substituent;
R³ represents a lower alkyl group with an optional substituent, a cycloalkyl group with an optional substituent, an aryl group with an optional substituent, or a heterocyclic group with an optional substituent;
R⁴ represents a hydrogen atom, a lower alkyl group with an optional substituent, an aryl group with an optional substituent, a heterocyclic group with an optional substituent, -OR⁵, -SR⁵ or -NR⁶R⁷ (wherein R⁵, R⁶ and R⁷ may be the same group or different groups, each represents a hydrogen atom, a lower alkyl group with an optional substituent, a cycloalkyl group with an optional substituent, an aryl group with an optional substituent, a heterocyclic group with an optional substituent, a lower alkoxy group, or an amino group with an optional substituent, and R⁶ and R⁷ may together form a group represented by -(CH₂)ₘ - or -(CH₂)ₗ NR⁸ (CH₂)ₖ - where k, 1, and m each represent an integer of 1 to 8 and R⁸ represents a hydrogen atom or a lower alkyl group);
X and Y may be the same group or different groups and each represents -CH₂-, -NH-, or -O-; and n represents an integer of 0 to 4.

The above compound represented by general formula (I) is preferably the compound of the following formula (an indolin-2-one derivative, Compound A stated below).

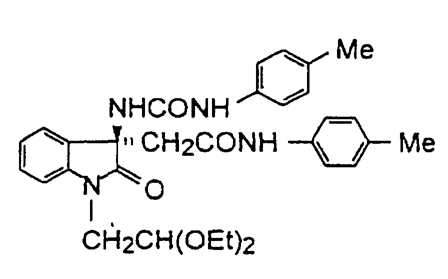

The present invention also provides a cartilage repair agent, a chondrocyte phenotype maintaining agent, a bone fracture repair promoter, and a therapeutic or prophylactic agent for a cartilage disease, each of which contains the foregoing chondrogenesis promoter as an active ingredient.

The present invention further provides an reagent for biological, physical, or chemical research on cartilage, each of which contains the foregoing chondrogenesis promoter.

The present invention also provides a method for promoting chondrogenesis comprising administering an effective amount of the foregoing chondrogenesis promoter to a patient, and a therapeutic or prophylactic method for cartilage disease comprising administering an effective amount of the foregoing chondrogenesis promoter to a patient.

The present invention further provides a low molecular weight compound having a chondrogenesis promoting effect for use in combination with an MEK1 inhibitor, wherein the low molecular weight compound comprises compound represented by aforementioned general formula (I) or a salt thereof as an active ingredient.

Here the compound represented by general formula (I) is preferably the aforementioned compound A.

The present invention also provides a therapeutic or prophylactic method for cartilage disease, comprising administering to a patient a low molecular weight compound having a chondrogenesis promoting effect and a compound with MEK1 inhibitory activity simultaneously, or within an interval in which the effect of combined use thereof can substantially appear.

In addition to the above, the present invention provides a kit comprising a first container for containing the foregoing low molecular weight compound having a chondrogenesis promoting effect, and a second container for containing a compound with MEK1 inhibitory activity; and also provides the kit further comprising instructions for administering to a patient the low molecular weight compound having a chondrogenesis promoting effect and the compound with MEK1 inhibitory activity.

The foregoing cartilage disease is, for example, osteoarthritis, chronic rheumatoid arthritis, osteochondritis dissecans, articular cartilage damage, herniated intervertebral disk, anotia, or microtia cartilage defect.

The present invention also provides a method for proliferating chondrocyte comprising culturing chondrocytes in the presence of the foregoing chondrogenesis promoter of the present invention, thereby obtaining proliferated chondrocytes; and also provides the method for proliferating chondrocyte wherein an extracellular matrices are resultantly formed along with proliferation of the chondrocyte to obtain chondrocytes with the extracellular matrices. In this case, the proliferated chondrocytes can be used for chondrocyte transplantation, and the chondrocyte transplantation includes autologous chondrocyte transplantation. The proliferated chondrocytes are preferably those twice or more as many as the original chondrocyte before the proliferation.

The present invention further provides a method for producing chondrocytes comprising culturing undifferentiated mesenchymal cells in the presence of the foregoing chondrogenesis promoter of the present invention to promote differentiation of the undifferentiated mesenchymal cell into chondrocytes, thereby obtaining chondrocytes; and also provides the method for producing chondrocyte wherein an extracellular matrices are resultantly formed along with differentiation of the undifferentiated mesenchymal cell into chondrocytes to obtain chondrocytes with the extracellular matrices. In this case, the chondrocytes obtained by promoting the differentiation of the undifferentiated mesenchymal cell into chondrocytes can be used for chondrocyte transplantation, and the chondrocyte transplantation includes autologous chondrocyte transplantation.

The present invention also provides a chondrocyte obtained by the foregoing method of the present invention for proliferating chondrocyte, and a chondrocyte obtained by the foregoing method of the present invention for producing chondrocyte.

The present invention further provides a chondrogenesis promoter of the present invention, wherein the chondrogenesis promoter has growth properties of chondrocytes, properties of extracellular matrix formation, or properties of differentiation of undifferentiated mesenchymal cells into chondrocytes.

### Brief Description of the Drawings

Fig. 1 is a drawing corresponding to a phase contrast photomicrograph showing rat articular chondrocytes after cultured with a solvent vehicle control (0.12% DMSO) for one week.
Fig. 2 is a drawing corresponding to a phase contrast photomicrograph showing rat articular chondrocytes after cultured with compound A (2 × 10⁻⁶ mol/L) for one week.
Fig. 3 is a drawing corresponding to a phase contrast photomicrograph showing rat articular chondrocytes after cultured with MEK1 inhibitor U0126 (1 × 10⁻⁵ mol/L) for one week.
Fig. 4 is a drawing corresponding to a phase contrast photomicrograph showing rat articular chondrocytes after cultured with compound A (2 × 10⁻⁶ mol/L) and MEK1 inhibitor U0126 (1 × 10⁻⁵ mol/L) for one week.
Fig. 5 is a graph showing amounts of the cartilage matrix in cell layers of rat articular chondrocytes after cultured with compound A (5 × 10⁻⁷ to 2 × 10⁻⁶ mol/L) for one week, in the presence or absence of MEK1 inhibitor U0126 (1 × 10⁻⁵ mol/L).
Fig. 6 is a graph showing amounts of the cartilage matrix in cell layers of rat articular chondrocytes after cultured with MEK1 inhibitor U0126 (1 × 10⁻⁷ to 1 × 10⁻⁵ mol/L) for one week, in the presence or absence of compound A (2 × 10⁻⁶ mol/L).
Fig. 7 is a drawing corresponding to a photomicrograph showing rat articular chondrocytes stained with alcian blue after cultured with a vehicle control (0.12% DMSO) or compound A (2 × 10⁻⁶ mol/L) or MEK1 inhibitor PD98059 (5 × 10⁻⁵ mol/L) or, compound A (2 × 10⁻⁶ mol/L) and MEK1 inhibitor PD98059 (5 × 10⁻⁵ mol/L) for one week.
Fig. 8 is a graph showing amounts of the cartilage matrix in cell layers of rat articular chondrocytes after cultured with MEK1 inhibitor PD98059 (2 × 10⁻⁶ to 5 × 10⁻⁵ mol/L) for one week, in the presence of compound A (2 × 10⁻⁶ mol/L).

### Best Mode for Carrying out the Invention

In the present invention, "having a chondrogenesis promoting effect" means having a capability of inducing differentiation of cartilage or a capability of enhancing the production of the cartilage matrix, and use of the term "having a chondrogenesis promoting effect" in "a low molecular weight compound having a chondrogenesis promoting effect" in the present invention means, particularly, having the capability of inducing differentiation of cartilage or the capability of enhancing the production of the cartilage matrix by some mechanism other than the MEK1 inhibitory action. The chondrogenesis promoting effect can be evaluated, for example, by measuring uptake of ³⁵S-labeled sulfuric acid in treatments of chondrocytes or cartilage precursor cells, utilizing the method described in Anal. Biochem. 81: 40-46, 1977. Specifically, it is preferable that the effect measured by the above measuring method is approximately two or more times greater than that in a control group.

In the present invention, "a low molecular weight compound having a chondrogenesis promoting effect" encompasses every low molecular weight compound having a such effect (which includes all compounds considered to be low molecular weight compounds in the field concerned and which includes, for example, organic compounds having the molecular weight of approximately 1000 or less); for example, potential compounds include indolin-2-one derivatives represented by aforementioned general formula (I) or salts thereof, low molecular weight organic compounds having a chondrogenesis promoting effect similar to that of these compounds, or 2-benzothiopyran and 3-benzothiepine derivatives and the like; among these compounds, preferred compounds are the indolin-2-one derivatives represented by foregoing general formula (I) or the salts thereof, and the low molecular weight organic compounds having the chondrogenesis promoting effect equivalent or similar to that of these compounds (whether a compound has "the chondrogenesis promoting effect equivalent or similar to" that of the indolin-2-one derivatives represented by foregoing general formula (I) can be determined, for example, by measuring the uptake of ³⁵S-labeled sulfuric acid as described above, or by measuring the production amount of sulfated glycosaminoglycans by the method described in Connective Tissue Res. 9: 247-248, 1982. For example, a compound can be determined to have the equivalent or similar effect when the compound demonstrates the effect comparable to or higher than that of the indolin-2-one derivatives by the above methods, i.e., the uptake of ³⁵S-labeled sulfuric acid or the production amount of sulfated glycosaminoglycans being 50% or more of those of the indolin-2-one derivatives represented by foregoing general formula (I) or the salts thereof); particularly, the low molecular weight compound is preferably selected from the indolin-2-one derivatives represented by foregoing general formula (I) or the salts thereof.

The compounds represented by general formula (I) are described in WO94/19322 and the official gazette thereof describes that the compounds are CCK-B/gastrin receptor antagonists. Furthermore, WO00/44722 describes that the compounds represented by general formula (I) have a chondrogenesis promoting effect.

The low molecular weight compound having a chondrogenesis promoting effect according to the present invention, and the compound with MEK1 inhibitory activity demonstrate synergistic effect in chondrogenesis promotion. Here the term "synergistic" means that the effect of combined use of two types of agents is greater than the sum of effects of the respective agents used singly.

The compound with MEK1 inhibitory activity is any compound having the effect of inhibiting MAPKK (Mitogen Activated Protein Kinase Kinase), lying upstream of p44/42MAPK in the MAPK (Mitogen Activated Protein Kinase) signaling pathway, i.e., MEK1; and the compounds with MEK1 inhibitory activity defined in the present invention encompasses all compounds having a such effect, including low molecular weight compounds, high molecular weight compounds, proteins, and the like. The MEK1 inhibitory activity can be measured by detecting phosphorylation of p44/42MAPK, for example, by the method described in J. Biol. Chem. 273: 18623-18632, 1998.

In the present invention, for example, compounds with inhibition activity being IC₅₀ of approximately 10 µmol/L are those demonstrating satisfactory synergistic effect with the low molecular weight organic compounds having a chondrogenesis promoting effect, and more preferred compounds are those with IC₅₀ of approximately 1 to 0.1 µmol/L. Specifically, the compounds with MEK1 inhibitory activity are, for example, U0126 (J. Biol. Chem. 273: 18623-18632, 1998), PD98059 (J. Biol. Chem. 270: 27489-27494, 1995), SL-327 (WO00/202097), BAY 43-9006 (Endocrine-Related Cancer 8: 219-225, 2001), and the like; these can be used singly or in combination of two or more compounds.

The cartilage repair agent is an agent that can be used for treatment of the cartilage diseases such as cartilage defect, osteoarthritis, and the like.

The chondrocyte phenotype maintaining agent is an agent which maintains the differentiation phenotype of chondrocytes, used as an adjuvant (culture medium additive) during in vitro cultivation of chondrocytes in the case of chondrocyte transplantation.

That is, the present invention provides the cartilage repair agent that can induce the cartilage differentiation of mesenchymal precursor cells, or promote the differentiation phenotype of mature chondrocytes. The present invention also provides the chondrocyte phenotype maintaining agent used as an adjuvant in chondrocyte transplantation, which can maintain the differentiation phenotype of mature chondrocytes. The present invention can provide a reagent having strong chondrogenesis promoting effect, which is useful to biological, physical, and chemical research on cartilage.

In the definition of the compound represented by general formula (I), the "halogen atom" refers to a fluorine atom, a chlorine atom, a bromine atom or an iodine atom.

The "lower alkyl group" is a linear or branched alkyl group of 1 to 6 carbons, such as a methyl group, an ethyl group, an n-propyl group, an i-propyl group, an n-butyl group, an s-butyl group, a t-butyl group, a pentyl group, a hexyl group, or the like.

The "lower alkenyl group" is a linear or branched alkenyl group of 2 to 6 carbons, such as a vinyl group, an allyl group, a butenyl group, a pentenyl group, a hexenyl group, or the like.

The "lower alkynyl group" is a linear or branched alkynyl group of 2 to 6 carbons, such as an ethynyl group, a propynyl group, a butynyl group, or the like.

The "lower alkoxy group" is a linear or branched alkoxy group of 1 to 6 carbons, such as a methoxy group, an ethoxy group, an n-propoxy group, an i-propoxy group, an n-butoxy group, an s-butoxy group, a t-butoxy group, a pentoxy group, a hexoxy group, or the like.

The "acyl group" is a carbonyl group that may have a hydrogen atom or with an optional substituent, such as an alkyl group, an aryl group, an alkoxy group, an amino group, or the like: examples of such groups include alkylcarbonyl groups such as an acetyl group, a propionyl group, a pivaloyl group, a cyclohexanecarbonyl group, or the like; arylcarbonyl groups such as a benzoyl group, a naphthoyl group, a toluoyl group, or the like.

The "aryl group" is a monovalent group obtained by removing a hydrogen atom from an aromatic hydrocarbon, such as a phenyl group, a tolyl group, a xylyl group, a biphenyl group, a naphthyl group, an anthryl group, a phenanthryl group, or the like.

The "lower alkylene group" is a linear or branched alkylene group of 1 to 6 carbons, such as a methylene group, an ethylene group, a propylene group, a butylene group, a pentylene group, a hexylene group, or the like.

The "cycloalkyl group" is a saturated cyclic hydrocarbon group of 3 to 8 carbons, such as a cyclopropyl group, a cyclobutyl group, a cyclopentyl group, a cyclohexyl group, a cycloheptyl group, or the like; and examples of the substituted cycloalkyl groups include a menthyl group, an adamantyl group, or the like.

The "heterocyclic group" is an aromatic heterocyclic group with at least one hetero atom, such as a pyridyl group, a furyl group, a thienyl group, an imidazolyl group, a pyrazinyl group, a pyrimidyl group, or the like.

The aforementioned lower alkyl group, lower alkenyl group, alkynyl group, lower alkoxy group, acyl group, aryl group, cycloalkyl group, and heterocyclic group may be optionally substituted with one or more selected from a variety of substituents. Examples of such substituents include a halogen atom, a lower alkyl group, a cycloalkyl group, an aryl group, a hydroxyl group, a lower alkoxy group which may be substituted with a halogen atom, an aryloxy group, a lower alkylthio group, a heterocyclic group, a formyl group which may be protected in the form of an acetal, a lower alkylcarbonyl group, an arylcarbonyl group, a carboxyl group, a lower alkoxycarbonyl group, an amino group which may have a lower alkyl group or the like, an imino group, a thioacetal group, a nitro group, a nitrile group, a trifluoromethyl group, or the like.

In the compound represented by general formula (I), R¹ is preferably a halogen atom, a lower alkyl group, a lower alkoxy group, or a nitro group.

The subscript "n" is preferably 0 or 1, and particularly, it is preferably 0.

R² is preferably a hydrogen atom, a lower alkyl group with an optional substituent, a lower alkenyl group with an optional substituent, or an aryl group with an optional substituent, and from the standpoint of activity as a chondrogenesis promoter or as a cartilage repair agent, it is preferably a lower alkyl group with an optional substituent (the substituent may be substituted with a halogen atom).

Among these, R² is more preferably a lower alkyl group substituted at the same carbon with two lower alkoxy groups (which may be substituted with 1 to 5 halogen atoms) or with the group -O-Z-O- wherein Z represents a lower alkylene group (which may be substituted with 1 to 10 halogen atoms), and is still more preferably a group represented by general formula (II) below: 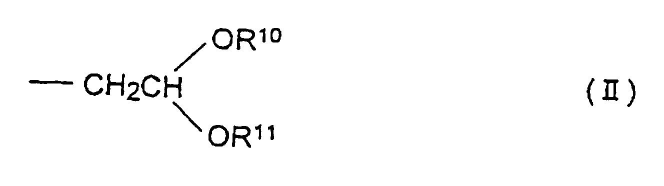 wherein
R¹⁰ and R¹¹ may be the same group or different groups, each represents a lower alkyl group optionally substituted with 1 to 5 halogen atoms, and, preferably, at least one of them is a lower alkyl group with 1 to 5 halogen atoms;
or a group represented by general formula (III) below: 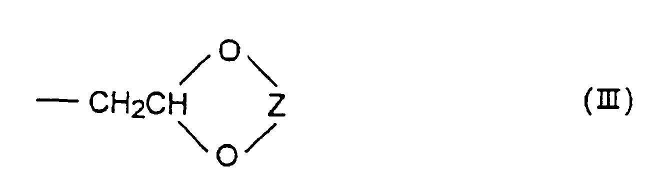 wherein Z represents a lower alkylene group optionally substituted with 1 to 10 halogen atoms.

Specifically, R² is preferably a 2,2-diethoxyethyl group, a 2,2-dimethoxyethyl group, a 2,2-diisopropoxyethyl group, a 2,2-bis(2-fluoroethoxy)ethyl group, or a 2,2-bis(2-chloroethoxy)ethyl group; among these, R² is more preferably a 2,2-diethoxyethyl group or a 2,2-bis(2-fluoroethoxy)ethyl group, and particularly preferably a 2,2-diethoxyethyl group.

R³ is preferably a lower alkyl group with an optional substituent, a cycloalkyl group with an optional substituent, or an aryl group with an optional substituent, and more preferably an aryl group with an optional substituent among them. The substituent is preferably a lower alkyl group (preferably a methyl group or an ethyl group; and more preferably a methyl group), or an amino group optionally substituted with a lower alkyl group; the group R³ is preferably a 4-methylphenyl group.

R⁴ is preferably a lower alkyl group with an optional substituent, an aryl group with an optional substituent, a heterocyclic group with an optional substituent, or a group represented by -OR⁵ or -NR⁶R⁷ wherein R⁵, R⁶ and R⁷ are as previously defined, and it is more preferably a group represented by -NR⁶R⁷ wherein R⁶ and R⁷ may be the same group or different groups and each represents a hydrogen atom or an aryl group with an optional substituent.

Among these, R⁴ is preferably a group represented by -NR⁶R⁷ wherein R⁶ and R⁷ may be the same or different groups and each represents a hydrogen atom or an aryl group (the aryl group has a lower alkyl group or an amino group which optionally substituted with a lower alkyl group), and it is particularly preferably a group represented by -NHR⁷ wherein R⁷ is a 4-methylphenyl group, a 4-ethylphenyl group, or a 4-(N,N-dimethylamino)phenyl group.

X is preferably -CH₂-, -NH-, or -O-.

Y is preferably -CH₂- or -NH-.

From the standpoint of activity as a chondrogenesis promoter, X and Y may be identical to or different from each other, and it is more preferable that X and Y are -CH₂- or -NH-, and particularly preferably, X is -NH- and Y - CH₂ -.

Specific examples of the compound represented by general formula (I) include the compounds mentioned in the examples of WO94/19322, i.e., the compound of Compound Nos. 1 to 201 listed in Tables 1 to 9 below. In Tables 1 to 9; R¹ to R⁴, X, Y, and n have the same definitions as those in general formula (I) above.

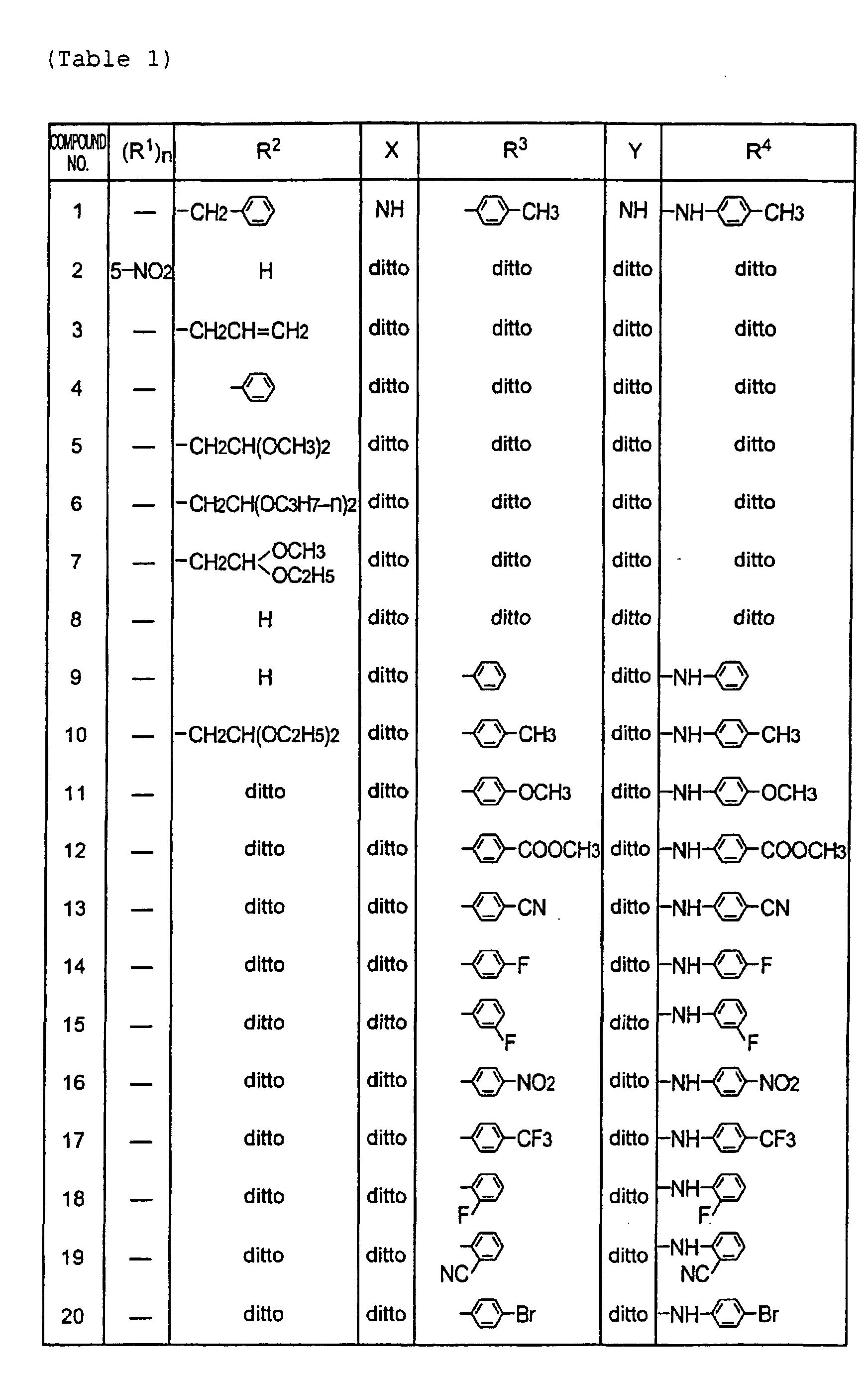

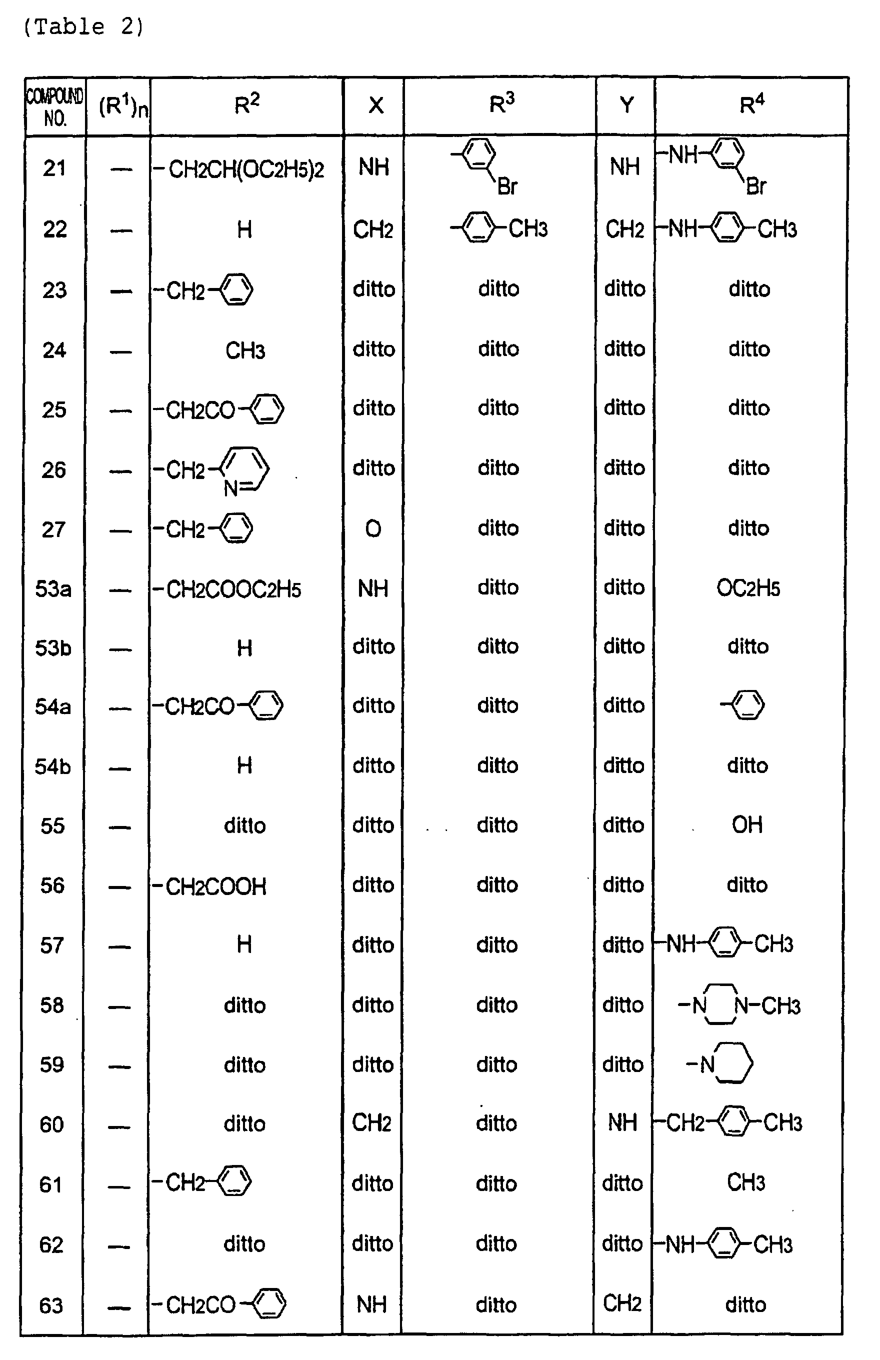

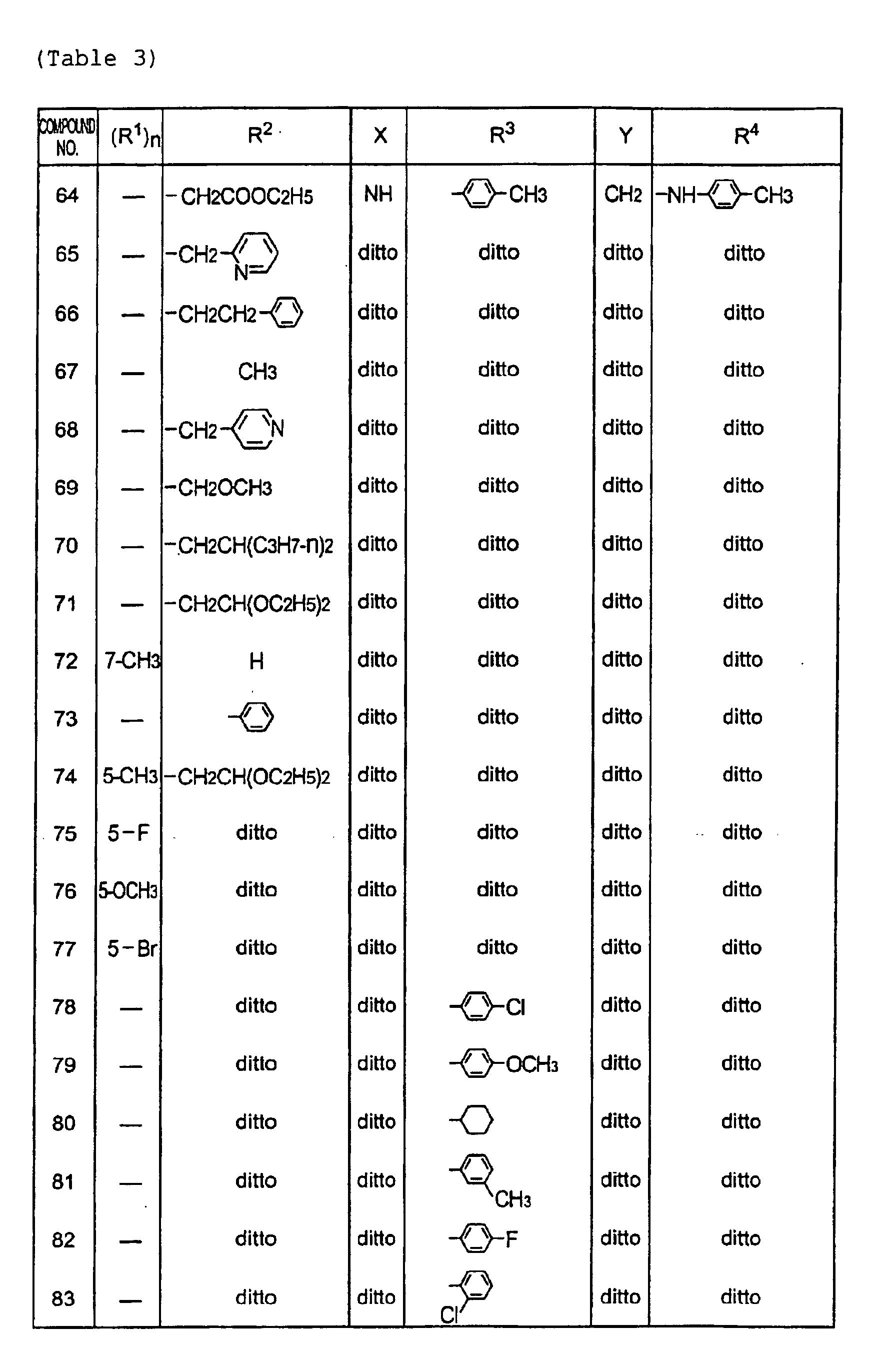

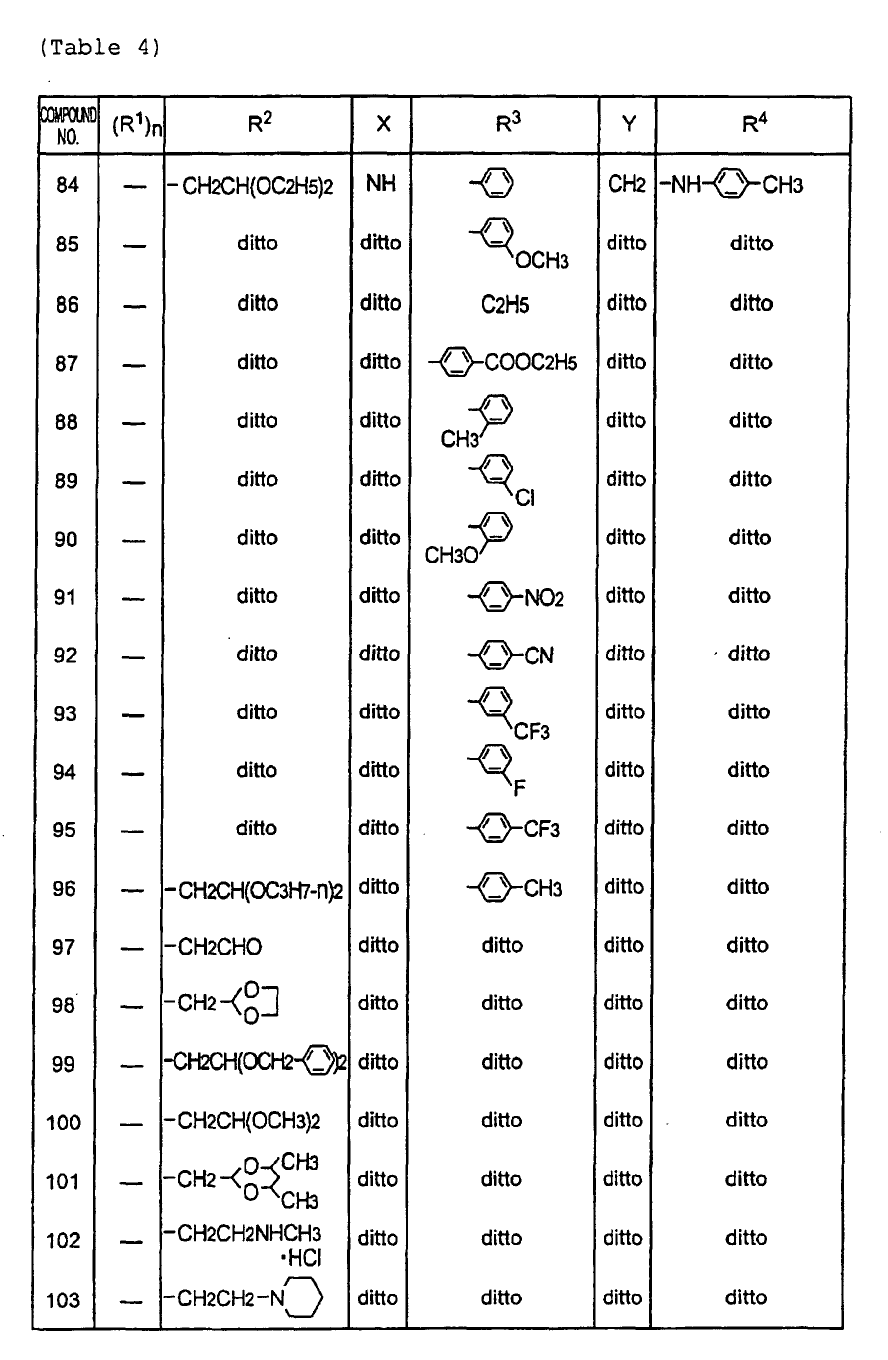

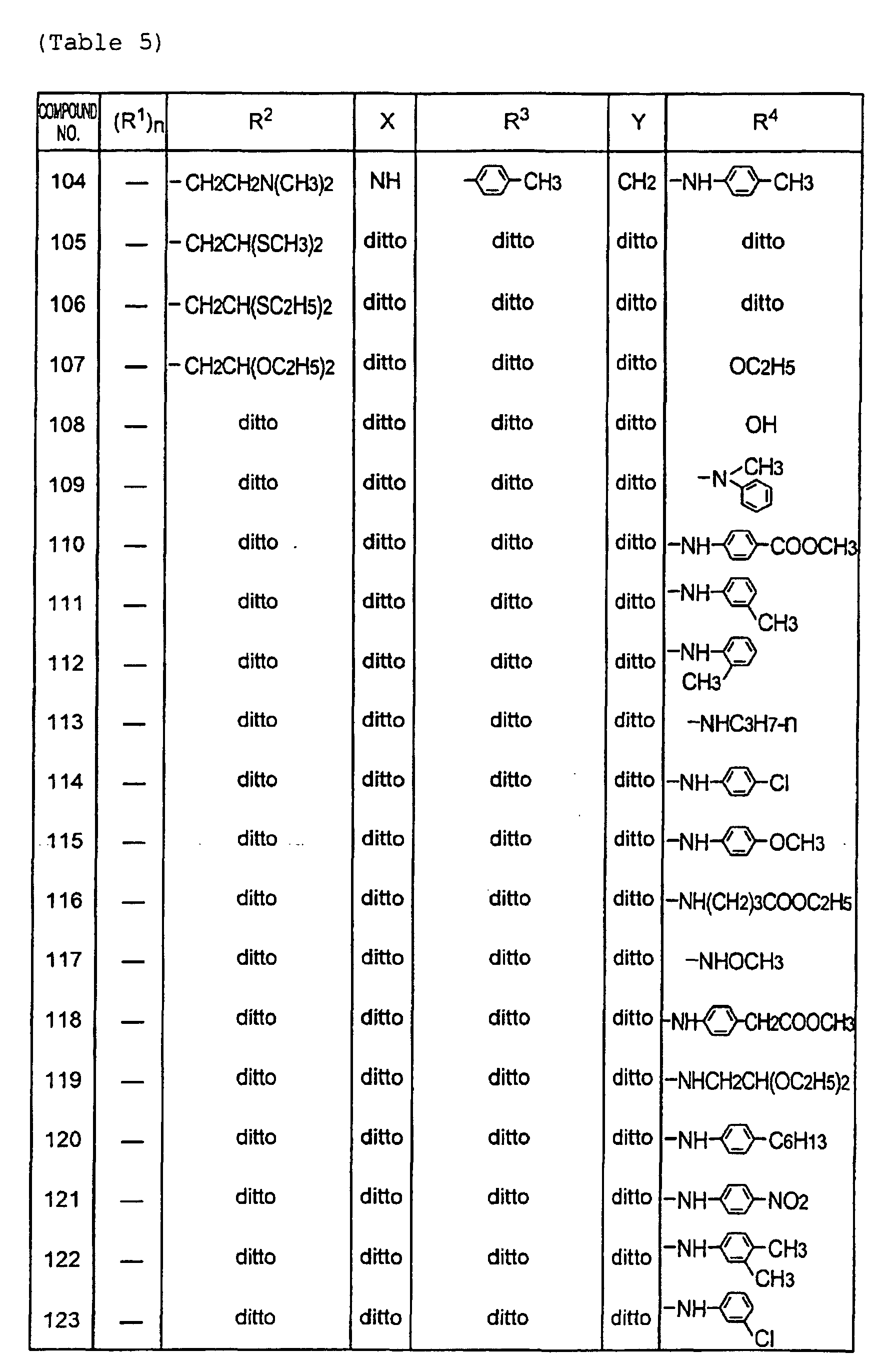

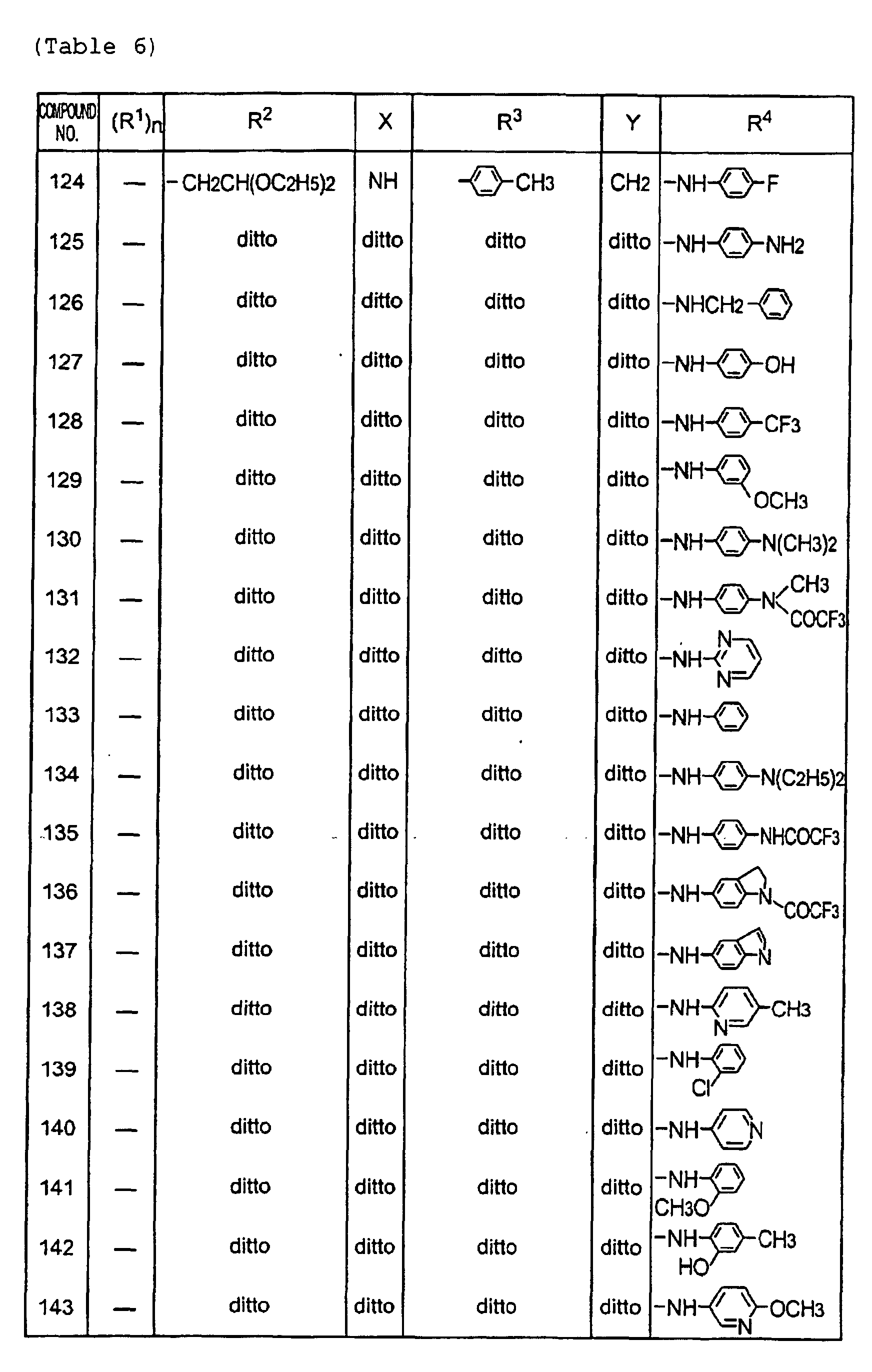

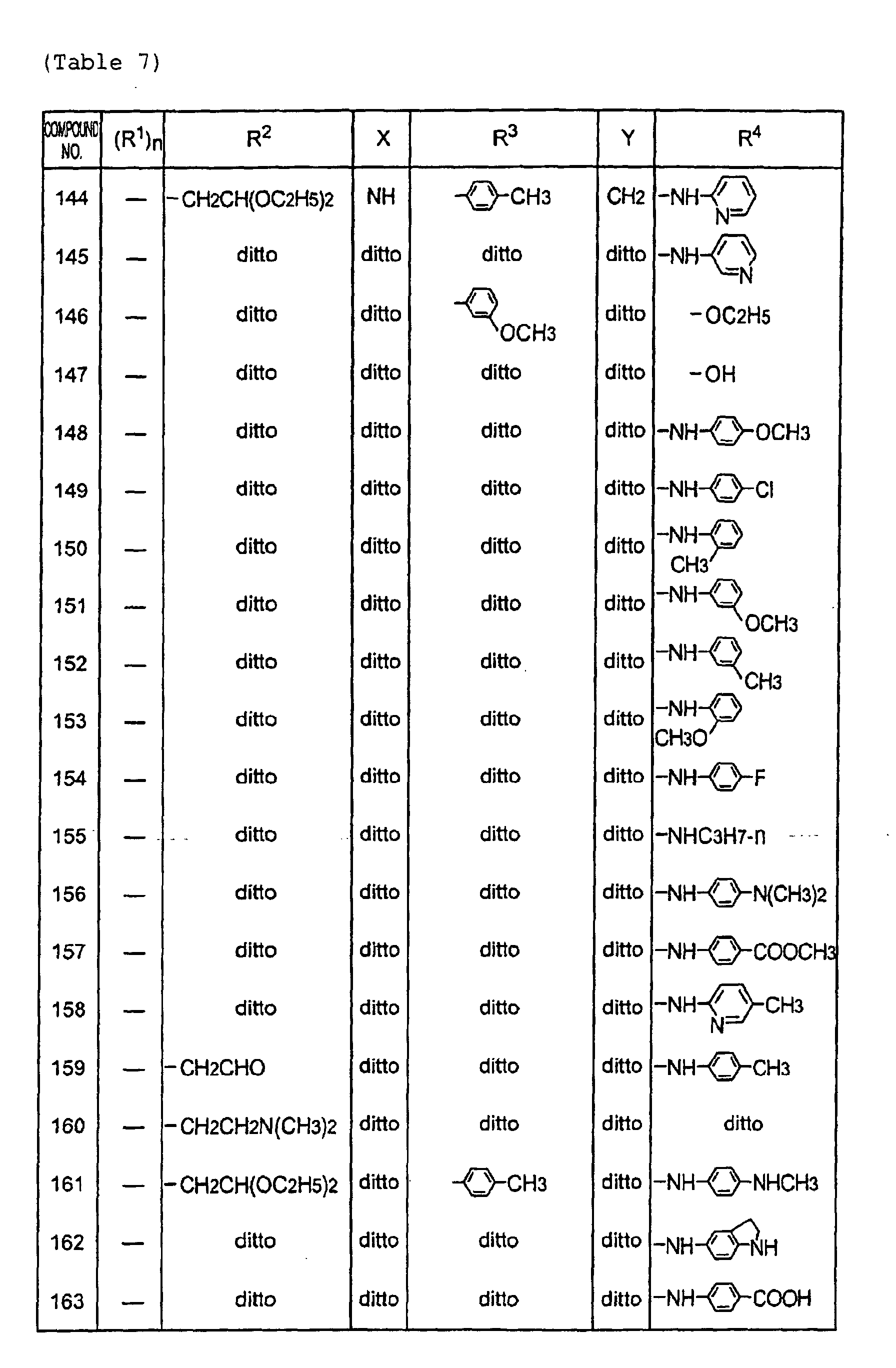

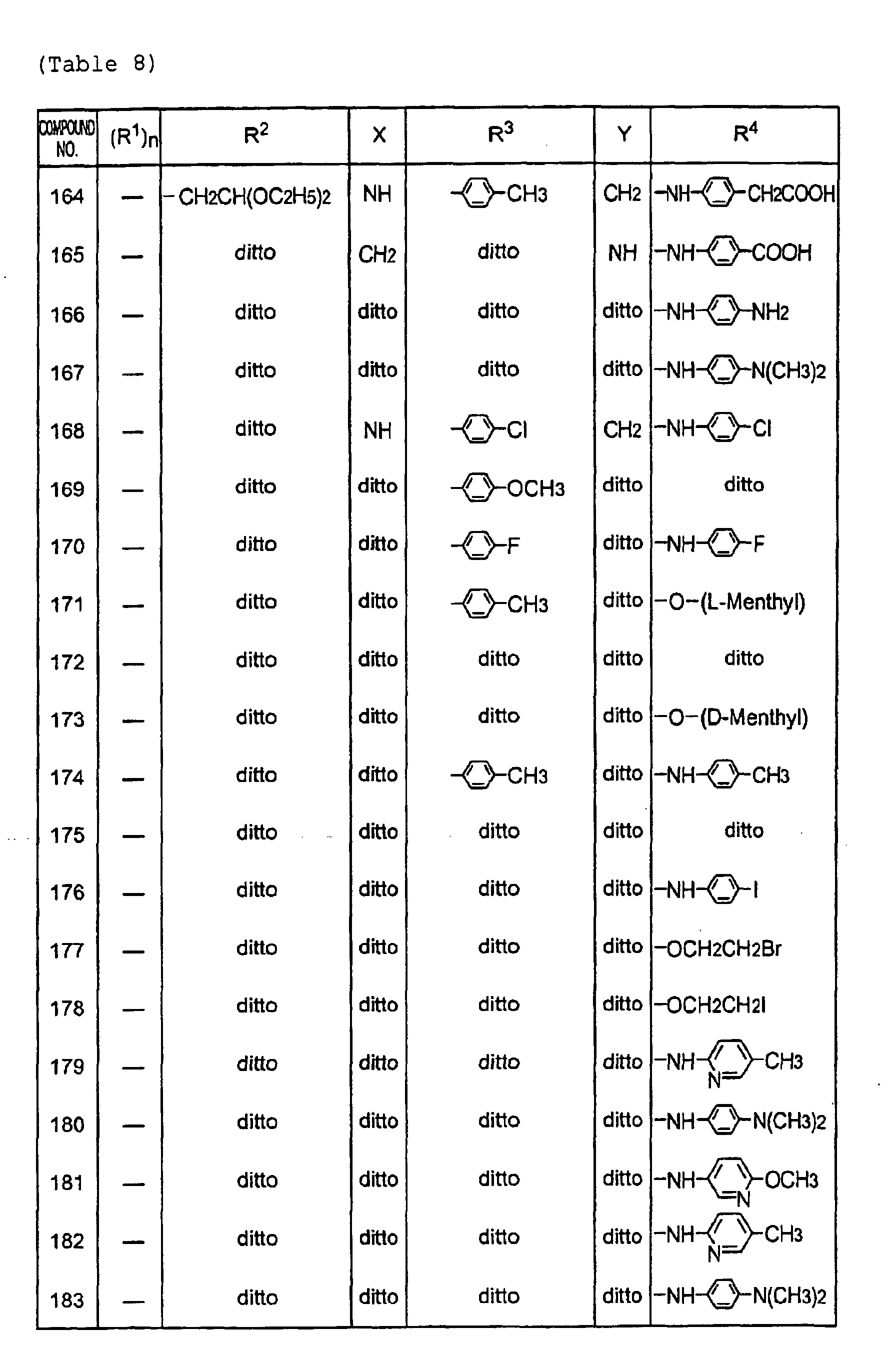

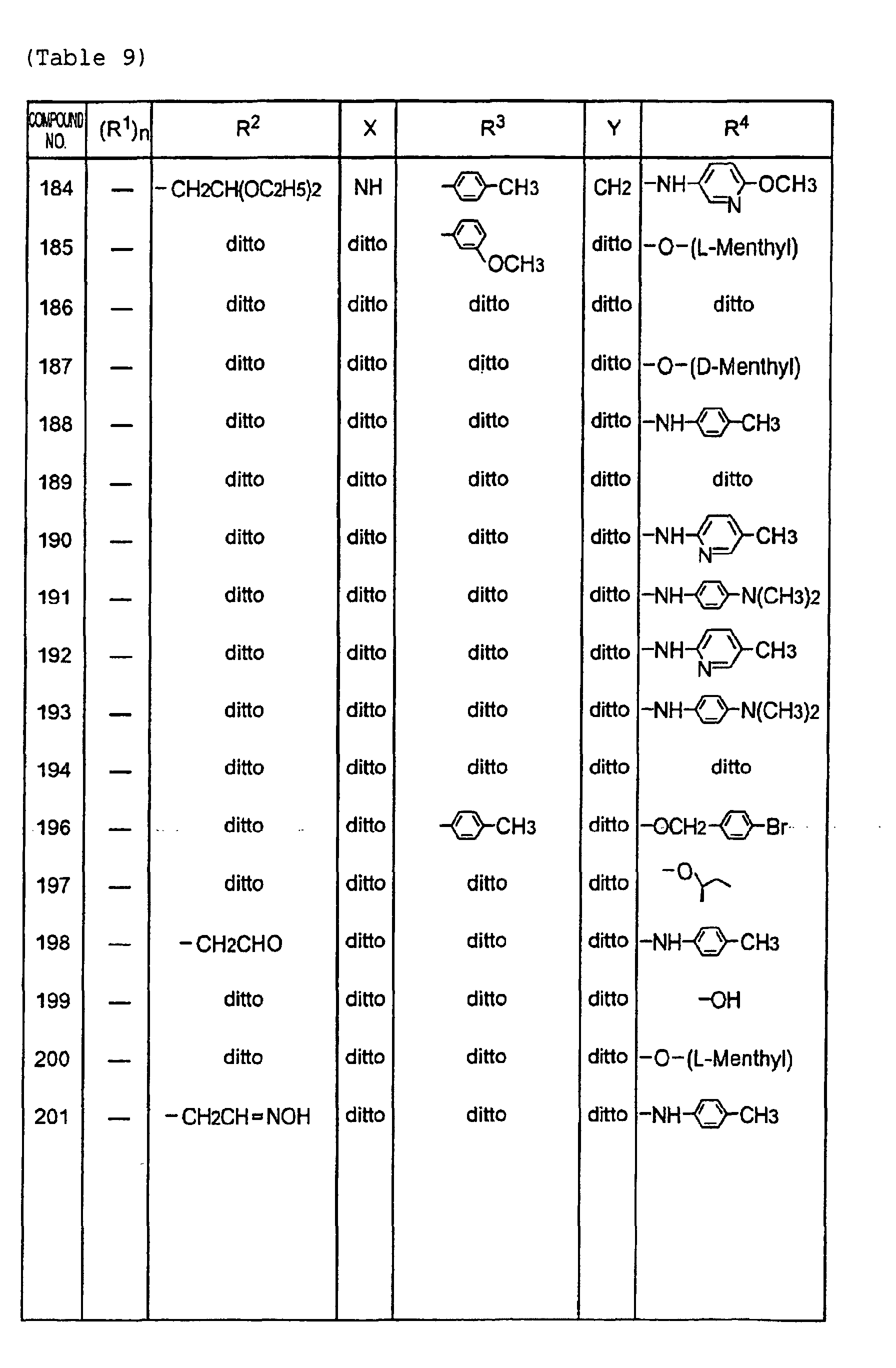

As the compound represented by general formula (I), the following Compounds A to G are preferred, and Compound A is particularly preferred.

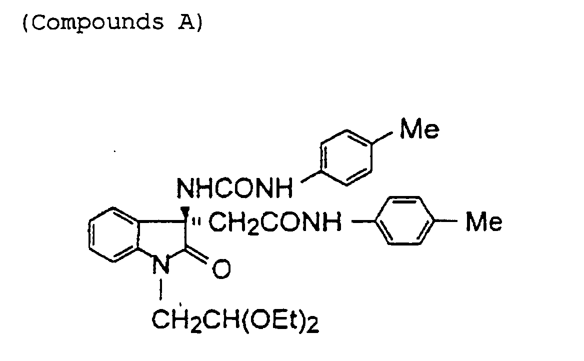

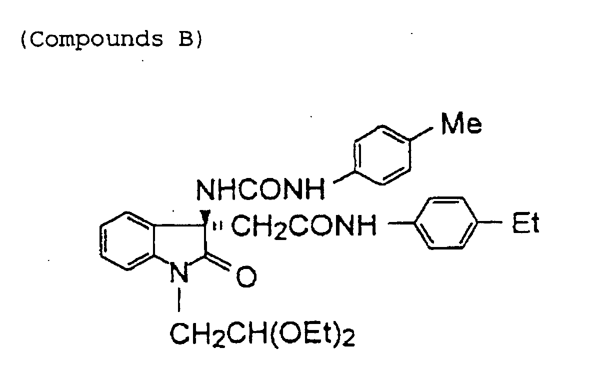

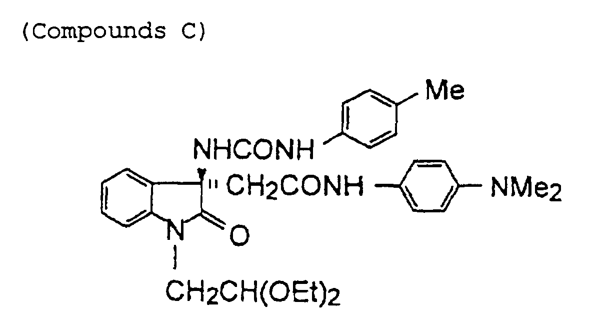

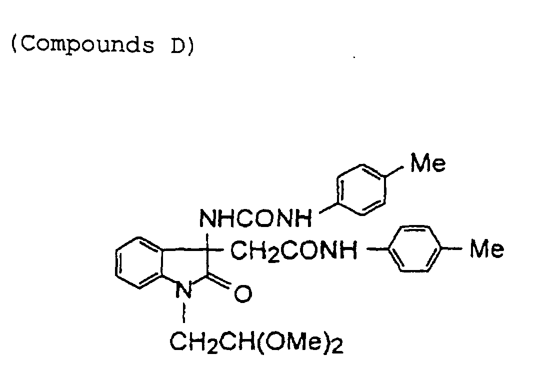

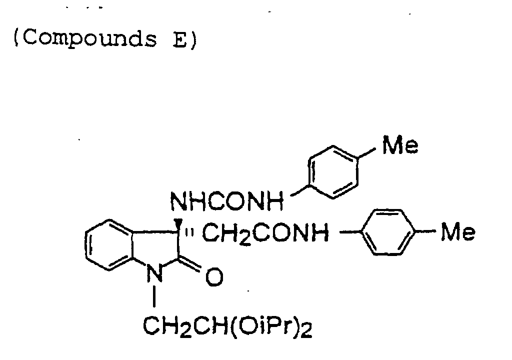

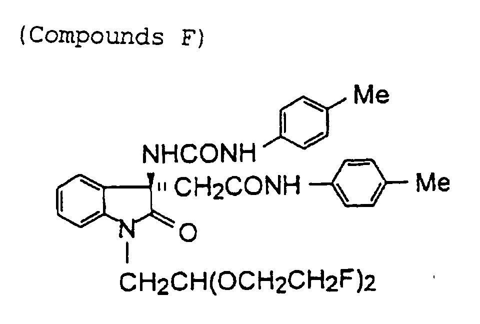

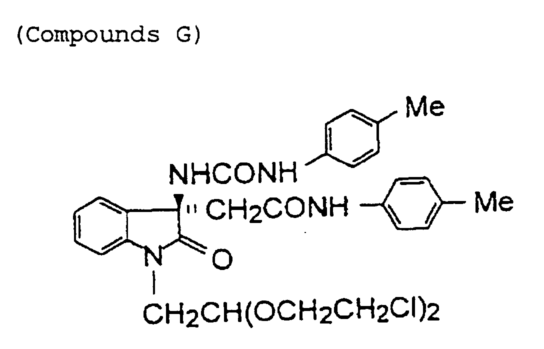

Examples of the salts of the compound represented by general formula (I) include inorganic salts such as hydrochloric acid salts, hydrobromic acid salts, hydroiodic acid salts, sulfuric acid salts, and phosphoric acid salts; organic acid salts such as succinic acid salts, malonic acid salts, acetic acid salts, maleic acid salts, fumaric acid salts, citric acid salts, benzoic acid salts, and salicylic acid salts; and metal salts such as sodium salts, potassium salts, and magnesium salts. Furthermore, the compound represented by general formula (I) may also be an optically active form. In the case of the compound is an optically active form, the absolute configuration at the 3 position is preferably the R configuration.

A chondrogenesis promoter according to the invention may be applied for a variety of uses without any particular restrictions, so long as the chondrogenesis promoting effect of the agent can be effectively utilized; it is particularly useful for treatment of chondropathy accompanying cartilage dysfunction due to degeneration or destruction of cartilage, treatment of damage or ablation of cartilage due to injury or surgery, or treatment of congenital cartilage hypoplasia or malformation. Furthermore, it is useful for inducing cartilage from undifferentiated precursor cells or for maintaining the phenotype of chondrocytes, in repair of cartilage defect by cell transplantation. Examples of such chondropathic conditions include osteoarthritis, chronic rheumatoid arthritis, osteochondritis dissecans, traumatic articular cartilage damage, herniated intervertebral disk, and the like. Examples of congenital cartilage hypoplasia and malformation include anotia, microtia, and the like.

An agent containing a compound according to the invention may be administered either orally or parenterally, but parenteral administration is preferred from the standpoint of avoiding unnecessary promotion of chondrogenesis outside of the site of administration, and from the standpoint of the effect. For the administration, the agent may be formulated in a manner suitable for the method of administration. The agent may also be used as a culture medium component in in vitro cultivation of undifferentiated precursor cells or chondrocytes, in the case of cell transplantation.

The chondrogenesis promoter of the present invention can be used as a cartilage repair agent, a chondrocyte phenotype maintaining agent, or a bone fracture repair promoter and can also be used as a therapeutic or prophylactic agent for a cartilage disease. In such applications, the agent may be administered by a method of making a composite agent by mixing the low molecular weight compound having a chondrogenesis promoting effect and the compound with MEK1 inhibitory activity before administration (including use in vitro; the same will also apply to the below) and then administering the composite agent, or by a method of simultaneously administering them without making the composite agent of the low molecular weight compound with a chondrogenesis promoting effect and the compound with MEK1 inhibitory activity.

Furthermore, the agent may be administered by a method of first administering one of the low molecular weight compounds with a chondrogenesis promoting effect and the compound with MEK1 inhibitory activity and thereafter administering the other. In this method, the compounds need to be administered within an interval in which the substantial effect of combined use thereof can appear. Here the interval in which the substantial effect of combined use can appear, may be properly determined according to elimination rates of the respective compounds in vivo. In the method of first administering one and then administering the other, the entire amount of either does not have to be administered at a time; for example, each of the low molecular weight compound having a chondrogenesis promoting effect and the compound with MEK1 inhibitory activity may be administered in several steps.

In order to implement the separate administration of the low molecular weight compound with a chondrogenesis promoting effect and the compound with MEK1 inhibitory activity, the present invention provides the low molecular weight compound having a chondrogenesis promoting effect, for use in combination with the compound with MEK1 inhibitory activity, wherein the low molecular weight compound comprises the compound represented by aforementioned general formula (I) or a salt thereof as an active ingredient. The compound represented by general formula (I) is preferably the aforementioned indolin-2-one derivative (the above compound A).

This enables implementation of a therapeutic or prophylactic method for a cartilage disease, comprising a step of administering to a patient the low molecular weight compound having a chondrogenesis promoting effect and the compound with MEK1 inhibitory activity simultaneously, or within an interval in which the effect of combined use thereof can substantially appear.

For implementing this method, the present invention provides a kit comprising a first container for containing the low molecular weight compound having a chondrogenesis promoting effect and a second container for containing the compound with MEK1 inhibitory activity. This kit may further comprise instructions for administering to a patient the low molecular weight compound having a chondrogenesis promoting effect and the compound with MEK1 inhibitory activity.

The first container and second container may be of the same type or of different types; for example, they may be injection syringes, vials, or press through packaging (PTP) containing tablets or capsules. The above instructions preferably include description of how to administrate the low molecular weight compound with a chondrogenesis promoting effect and the compound with MEK1 inhibitory activity, the interval for appearance of the substantial effect of combined use, and the like.

In the present invention, the combined use of the low molecular weight compound having a chondrogenesis promoting effect and the compound with MEK1 inhibitory activity exhibits the synergistic chondrogenesis promoting effect. Since the low molecular weight compound having a chondrogenesis promoting effect and the compound with MEK1 inhibitory activity can be used separately as described above, the compounds may be prepared as separate products, or may be mixed with each other to form a single product. A mode of administration is, for example, a method of separately preparing these compounds and administering them by a method similar to that in respective clinical use of them by which the desired effect can be expected at smaller amounts than when used singly.

A pharmaceutical composition comprising the compound having a chondrogenesis promoting effect and the compound with MEK1 inhibitory activity may be formulated using an ordinary formulation technique; and may be used in various forms depending on the purpose of use, such as in the form of capsules, granules, cream, powder, syrup, tablets, injection or ointment, either in solid or liquid form. The carrier or excipient used for the formulation may be a solid or liquid substance. As examples there may be mentioned lactose, magnesium stearate, starch, talc, gelatin, agar, pectin, gum Arabic, olive oil, sesame oil, ethylene glycol, polylactic acid (PLA), polyglycolic acid (PGA), a copolymer of polylactic acid and polyglycolic acid (PLGA), hyaluronic acid, collagen sponge, and the like, as well as any other commonly used substance.

The content of the chondrogenesis promoter in the pharmaceutical composition will differ depending on the form of preparation, and in general, the pharmaceutical composition preferably contains the low molecular weight compound having a chondrogenesis promoting effect in a concentration of 0.00001 to 80 wt % and the compound with MEK1 inhibitory activity in a concentration of 0.00001 to 80 wt%. The weight ratio (or the ratio of administration amounts) of the low molecular weight compound with a chondrogenesis promoting effect to the compound with MEK1 inhibitory activity can be altered in a wide range according to types of diseases, ages, the severity of symptoms, administration routes, doctor's diagnosis, and the like as long as the synergistic effect according to the invention can be expected; in general, the compounds can be used in the range in which the ratio of the low molecular weight compound having a chondrogenesis promoting effect : the compound with MEK1 inhibitory activity ranges from 1000:1 to 1:100. For example, when they are used as culture medium additives in an in vitro culturing system in autologous chondrogenesis transplantation, the low molecular weight compound with a chondrogenesis promoting effect can be used in the range of 0.1 to 10 µmol/L and the compound with MEK1 inhibitory activity in the range of 0.01 to 10 µmol/L.

In the case of the oral administration, generally, the low molecular weight compound having a chondrogenesis promoting effect is used in the amount ranging from 0.01 to 2 g/kg per day and the compound with MEK1 inhibitory activity also in the amount ranging from 0.01 to 2 g/kg per day; in the case of the parenteral administration, generally, the low molecular weight compound having a chondrogenesis promoting effect is used in the amount ranging from 0.0000001 to 0.01 g/kg per day, and the compound with MEK1 inhibitory activity in the amount ranging from 0.0000001 to 0.01 g/kg per day. The above dosages may be given together or separately once or several times every 1 to 7 days, and the way of administration can be adjusted depending on the severity of symptoms and the doctor's diagnosis.

As shown in the examples provided below, it was confirmed that excellent effect of promoting production of cartilage matrix was achieved when the agents according to the invention, the low molecular weight compound having a chondrogenesis promoting effect (particularly, the compound represented by general formula (I)) and the compound with MEK1 inhibitory activity, were added to articular chondrocytes obtained from rats. This fact verifies the usefulness of the agents comprising the low molecular weight compound including the compound represented by general formula (I) or a compound showing the effect similar thereto and the compound with MEK1 inhibitory activity, as treatment agents for articular diseases; and the usefulness of the medical treatment for articular diseases with the combined use of these compounds.

When the rat articular chondrocytes were treated with the chondrogenesis promoter containing the low molecular weight compound having a chondrogenesis promoting effect (e.g., the compound represented by general formula (I) or any compound having a effect similar thereto) and the compound with MEK1 inhibitory activity, or treated with a combination of these compounds, we successfully obtained chondrocytes (an increased number of chondrocytes after treatment of original chondrocytes), an extracellular matrix of the chondrocytes, or cartilage-like tissue. This proves that these chondrogenesis promoters can be effective treatment agents as repair agents for defect of articular cartilage, tracheal cartilage, intervertebral disks, auricular cartilage, sternal cartilage, and the like.

The present effect also indicates that the chondrogenesis promoter containing the low molecular weight compound having a chondrogenesis promoting effect (e.g., the compound represented by general formula (I) or any compound with the effect similar thereto) and the compound with MEK1 inhibitory activity is useful as a chondrocyte phenotype maintaining agent during repair of cartilage defect by chondrocyte transplantation. Furthermore, the chondrogenesis promoter containing the low molecular weight compound including the compound represented by general formula (I) or any compound with the effect similar thereto and the compound with MEK1 inhibitory activity can be used for analysis of extracellular matrix metabolism of chondrocytes and the mechanism of differentiation into chondrocytes (biological properties), analysis of the components constituting the extracellular matrix (chemical properties), and analysis of properties such as viscoelasticity (physical properties).

Furthermore, the agent of the present invention, which contains the compound having the strong chondrogenesis promoting effect represented by general formula (I) or any other compound with the effect similar thereto, and the compound with MEK1 inhibitory activity, can promote formation of calcified cartilage by the chondrogenesis promoting effect, and, in that case, we can eventually expect a bone fracture repair promoting effect via endochondral ossification with the chondrogenesis promoter. That is, the chondrogenesis promoter can also be expected to be useful as a bone fracture repair promoter.

The present invention will now be explained in more concrete detail by way of the following examples. In the description hereinafter "parts" and "%" indicating amount ratios will be described on a weight basis unless otherwise stated.

### (Example 1: synergistic cartilage matrix production promoting effect of compound A and compound with MEK1 inhibitory activity U0126 in rat articular chondrocytes)

A 5-week-old male SD rat (Charles River Japan Corp.) was euthanized, and thereafter the femur was removed therefrom, and the articular cartilage layer was stripped off from the knee joint surface of the femur with a scalpel. The articular cartilage obtained was treated with 0.15% trypsin (DIFCO Lab.)/0.05% EDTA (KANTO KAGAKU CORP.) at 37°C for one hour, and thereafter the matrix was decomposed while being agitated at 37°C in 0.15% collagenase (WORTHINGTON Corp.) for three hours, thereby obtaining a cell suspension. The cartilage cells obtained were seeded at the cell density of 15000 per well in a 24-well culturing plate (Falcon Corp.) coated with type I collagen (Nitta Gelatin Inc.) and were cultured in a Dulbecco's modified Eagle medium (Gibco) containing 10% fetal bovine serum (REHATUIN) and antibiotic-antimycotic (Gibco) (Calcif. Tissue Int. 19: 179-187, 1975). After achievement of the confluent state of cells, 50 µg/mL ascorbic acid (Wako Pure Chemical Industries) was added to the medium, and either or both of 5×10⁻⁷ to 2×10⁻⁶ mol/L compound A and 10⁻⁷ to 10⁻⁵ mol/L compound with MEK1 inhibitory activity U0126 (Promega) were further added thereto, followed by culture for one week. 0.12% DMSO (Wako Pure Chemical Industries) was used as a solvent vehicle control. The medium was replaced every two to three days and the above agents were added every replacement.

After completion of the culture, the cell layer was fixed at room temperature in 4% paraformaldehyde solution (Wako Pure Chemical Industries) for two hours and then washed with distilled water and 5% acetic acid of pH 1.0, and thereafter the cartilage matrix was stained at room temperature with alcian blue of pH 1.0 (Wako Pure Chemical Industries) for two hours. The cell layer was washed with 5% acetic acid and distilled water and then dried, 6 mol/L guanidine hydrochloride (Nacalai Tesque, Inc.) was added to the cell layer to extract the dye therefrom, and the absorption was measured at 630 nm with a Microplate reader (BioRad).

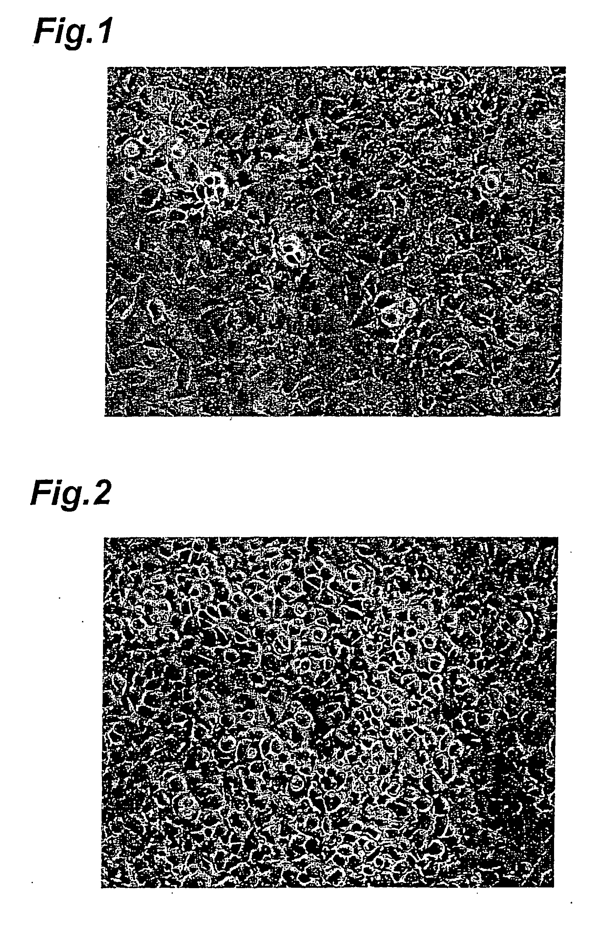

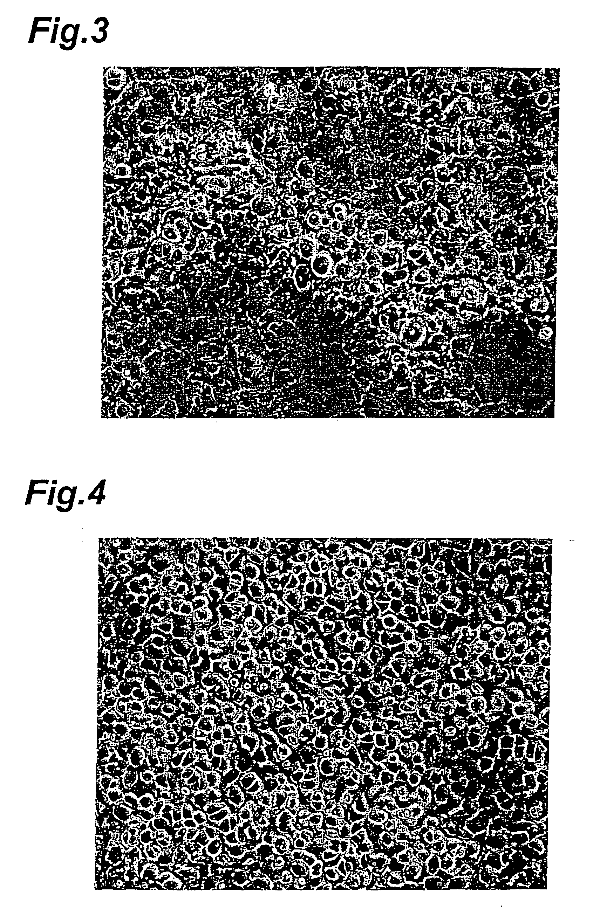

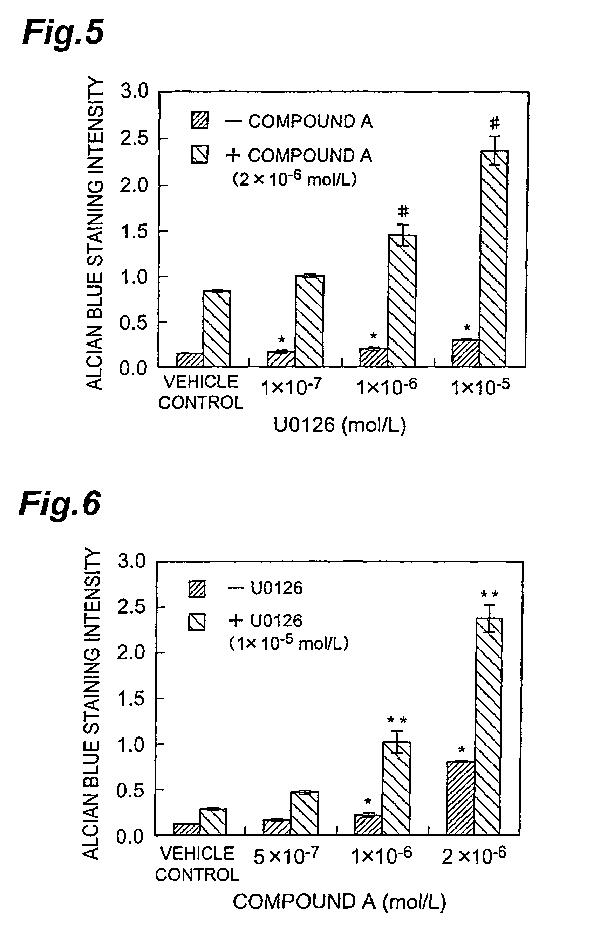

Morphologies of cells at the time of the end of the culture (phase contrast photomicrograph) are presented in Figs. 1 to 4. As apparent from the results of Figs. 1 to 4, in contrast to the vehicle control (Fig. 1), production of the matrix was enhanced in the chondrocytes cultured with compound A and the morphology of the chondrocytes changed to a round form (Fig. 2). The differentiation of chondrocytes was slightly enhanced even with the compound with MEK1 inhibitory activity U0126 alone (Fig. 3), and under the coexisting condition of the compound A and U0126, the production of the matrix was dramatically enhanced, so as to exhibit the morphology like typical mature chondrocytes with a clearly expanded clearance between cells (Fig. 4), as compared with the control group.

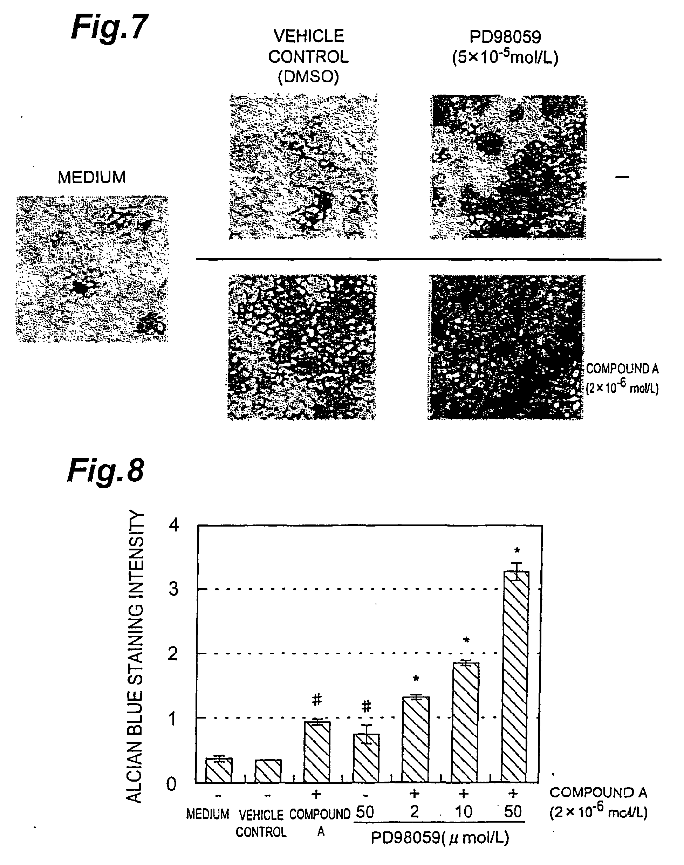

Alcian blue staining intensities are presented in Figs. 5 and 6. Fig. 5 shows the results of an assay without compound A nor U0126 but with only DMSO, assays without compound A but with U0126 in the respective concentrations of 1 × 10⁻⁷, 1 × 10⁻⁶, and 1 × 10⁻⁵ mol/L, an assay with only compound A in the concentration of 2 × 10⁻⁶ mol/L, assays with compound A in the concentration of 2 × 10⁻⁶ mol/L and with U0126 in the respective concentrations of 1 × 10⁻⁷, 1 × 10⁻⁶, and 1 × 10⁻⁵ mol/L. The figure shows average with ± standard deviation of alcian blue staining intensities of three wells for each group.

Fig. 6 shows the results of an assay without compound A nor U0126 but with DMSO, assays without U0126 but with compound A in the respective concentrations of 5 × 10⁻⁷, 1 × 10⁻⁶, and 2 × 10⁻⁶ mol/L, an assay with only U0126 in the concentration of 1 × 10⁻⁵ mol/L, and assays with U0126 in the concentration of 1 × 10⁻⁵ mol/L and with compound A in the respective concentrations of 5 × 10⁻⁷, 1 × 10⁻⁶, and 2 × 10⁻⁶ mol/L. The figure shows average with ±standard deviation of alcian blue staining intensities of three wells for each group.

Dunnett multiple-comparisons two-sided tests were conducted to verify whether the sole treatments with U0126 or with compound A had a significant difference in alcian blue staining intensity from the vehicle control, and whether the coexistence of the two agents demonstrated a significant difference in alcian blue staining intensity from each of the sole treatments.

In Figs. 5 and 6 mark * represents that each of U0126 and compound A in each concentration singly significantly (p < 0.05) enhanced the production of the alcian-blue-positive cartilage matrix as compared to the vehicle control. In Fig. 5 mark # represents that U0126 in each concentration in the presence of 2 × 10⁻⁶ mol/L compound A significantly (p < 0.05) enhanced the production of the alcian-blue-positive cartilage matrix as compared to the sole treatment with compound A. In Fig. 6 mark ** represents that compound A in each concentration in the presence of 1 × 10⁻⁵ mol/L U0126 significantly (p < 0.05) enhanced the production of the alcian-blue-positive cartilage matrix as compared to the sole treatment with U0126.

It is clear from the results in Figs. 5 and 6 that even compound A or U0126 alone significantly promoted the production of the cartilage matrix and that the two compounds under the coexisting condition demonstrated the synergistic effect of promoting the production of the cartilage matrix.

### (Example 2: synergistic cartilage matrix production promoting effect of compound A and compound with MEK1 inhibitory activity PD98059 in rat articular chondrocytes)

A 5-week-old male SD rat (Charles River Japan Corp.) was euthanized, and thereafter the femur was removed therefrom, and the articular cartilage layer was stripped off from the knee joint surface of the femur with a scalpel. The articular cartilage obtained was treated with 0.15% trypsin (DIFCO Lab.)/0.05% EDTA (Nacalai Tesque, Inc.) at 37°C for one hour, and thereafter the matrix was decomposed while being agitated at 37°C in 0.15% collagenase (WORTHINGTON Corp.) for three hours, thereby obtaining a cell suspension. The cartilage cells obtained were seeded at the cell density of 20000 per well in a 24-well culturing plate (Falcon Corp.) coated with type I collagen (Nitta Gelatin Inc.) and were cultured in a Dulbecco's modified Eagle medium (Gibco) containing 10% fetal bovine serum (REHATUIN) and antibiotic-antimycotic (Gibco) (Calcif. Tissue Int. 19: 179-187, 1975). After achievement of the confluent state of cells, 50 µg/mL ascorbic acid (Wako Pure Chemical Industries) was added to the medium, and either or both of 2×10⁻⁶ mol/L compound A and 2×10⁻⁶ to 5×10⁻⁵ mol/L compound with MEK1 inhibitory activity PD98059 (New England Biolab) were further added thereto, followed by culture for one week. 0.12% DMSO (JUNSEI CHEMICAL CO., LTD) was used as a solvent vehicle control. The medium was replaced every two to three days and the above agents were added every replacement.

After completion of the culture, the cell layer was fixed at room temperature in 4% paraformaldehyde solution (Wako Pure Chemical Industries) for two hours and then washed with distilled water and 5% acetic acid of pH 1.0, and thereafter the cartilage matrix was stained at room temperature with alcian blue of pH 1.0 (Wako Pure Chemical Industries) for two hours. The cell layer was washed with 5% acetic acid and distilled water and then dried, 6 mol/L guanidine hydrochloride (Nacalai Tesque, Inc.) was added to the cell layer to extract the dye therefrom, and absorption was measured at 595 nm with the Microplate reader (BioRad).

Morphologies of cells (phase contrast photomicrograph) after stained with alcian blue are presented in Fig. 7. As shown in Fig. 7, when cells were cultured with either compound A or PD98059, the ratio of alcian blue positive cells increased as compared to the vehicle control; and almost all cells were alcian blue positive in the case of compound A and PD98059 coexisting.

Fig. 8 shows alcian blue staining intensities. Fig. 8 shows the results of an assay without compound A nor PD98059 (medium), an assay without compound A nor PD98059 but with DMSO, an assay with only compound A in the concentration of 2 × 10⁻⁶ mol/L, an assay with only PD98059 in the concentration of 5 × 10⁻⁵ mol/L, and assays with compound A in the concentration of 2 × 10⁻⁶ mol/L and with PD98059 in the respective concentrations of 2 × 10⁻⁶, 1 × 10⁻⁵, and 5 × 10⁻⁵ mol/L. The figure shows average with ± standard deviation of alcian blue staining intensities of three wells for each group.

Dunnett multiple-comparisons two-sided tests were conducted to verify whether the sole treatments with PD98059 or with compound A had a significant difference in alcian blue staining intensity from the vehicle control and whether the coexistence of the two agents demonstrated a significant difference in alcian blue staining intensity from each of the sole treatments.

In Fig. 8 mark + on the horizontal axis represents the presence of compound A (2 × 10⁻⁶ mol/L), while mark - represents the absence of compound A. In the same figure mark # represents that each of PD98059 in each concentration and compound A singly significantly (unpaired t-test, p < 0.05) promoted the production of the alcian-blue-positive cartilage matrix as compared to the vehicle control. In the same figure mark * represents that PD98059 in each concentration in the presence of 2 × 10⁻⁶ mol/L compound A significantly (p < 0.05) promoted the production of the alcian-blue-positive cartilage matrix as compared to the sole treatment with compound A.

It is clear from Fig. 8 that even compound A or PD98059 alone significantly promoted the production of the cartilage matrix, and that the two compounds under the coexisting condition demonstrated the synergistic effect of promoting the production of the cartilage matrix.

These results confirmed that the compound A and the compound with MEK1 inhibitory activity had the effect of synergistically increasing the production of the cartilage matrix. Accordingly, the agents, which contains the low molecular weight compound including the chondrogenesis promoter represented by general formula (I) or any other compound with the effect similar thereto and the compound with MEK1 inhibitory activity, can be used as agents for osteoarthritis, chronic rheumatoid arthritis, osteochondritis dissecans, and repair of damage of articular cartilage due to injury, and also as agents used in chondrocyte transplantation, such as autologous chondrocyte transplantation, for promoting the capability of forming the extracellular matrix of chondrocytes or the capability of proliferating chondrocytes before transplantation and after transplantation.

### Industrial Applicability

The present invention provides the chondrogenesis promoter with the unprecedented strong effect. The invention also provides the cartilage repair agent, the chondrocyte phenotype maintaining agent, the bone fracture repair promoter, the therapeutic or prophylactic agent for the cartilage diseases, the reagent for biological, physical, or chemical research on cartilage, and the agent for chondrocyte transplantation, each of which contains the foregoing chondrogenesis promoter. Furthermore, the invention provides the chondrogenesis promoting method and the therapeutic or prophylactic method for the cartilage diseases, utilizing the foregoing chondrogenesis promoter. In addition, the invention provides the low molecular weight compound as a component of the foregoing chondrogenesis promoter, the therapeutic or prophylactic method for the cartilage diseases using the low molecular weight compound, and the kit for the foregoing chondrogenesis promoter. Furthermore, the object of the present invention was achieved to provide the chondrocyte proliferation method and production method utilizing the foregoing chondrogenesis promoter, and the chondrocytes obtained by these methods.

Potential applications of the chondrogenesis promoter of the present invention include excellent curative medicines for rheumatism, osteoarthritis, or chondropathy such as damage of cartilage due to injury, with promotion of the chondrogenesis in the warm-blooded animals including humans, and excellent phenotype maintaining agents for chondrocytes in autologous chondrocyte transplantation.

## Claims

1. A chondrogenesis promoter comprising a low molecular weight compound having a chondrogenesis promoting effect and a compound with MEK1 inhibitory activity.

2. A chondrogenesis promoter according to claim 1, wherein the low molecular weight compound having a chondrogenesis promoting effect is a compound represented by general formula (I) or a salt thereof: 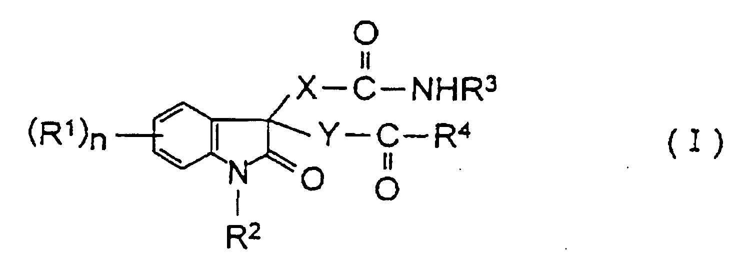 wherein
R¹ represents a halogen atom, a lower alkyl group, a lower alkoxy group, a hydroxyl group, a nitro group, a trifluoromethyl group, a lower alkylthio group, an acyl group, a carboxyl group, a mercapto group, or an amino group with an optional substituent;
R² represents a hydrogen atom, a lower alkyl group with an optional substituent, a lower alkenyl group with an optional substituent, a lower alkynyl group with an optional substituent, a lower alkoxy group with an optional substituent, an acyl group with an optional substituent, an aryl group with an optional substituent, or a heterocyclic group with an optional substituent;
R³ represents a lower alkyl group with an optional substituent, a cycloalkyl group with an optional substituent, an aryl group with an optional substituent, or a heterocyclic group with an optional substituent;
R⁴ represents a hydrogen atom, a lower alkyl group with an optional substituent, an aryl group with an optional substituent, a heterocyclic group with an optional substituent, -OR⁵, -SR⁵ or -NR⁶R⁷ (wherein R⁵, R⁶ and R⁷ may be the same or different and each represents a hydrogen atom, a lower alkyl group with an optional substituent, a cycloalkyl group with an optional substituent, an aryl group with an optional substituent, a heterocyclic group with an optional substituent, a lower alkoxy group or an amino group with an optional substituent, and R⁶ and R⁷ may together form a group represented by -(CH₂)ₘ- or -(CH₂)ₗNR⁸(CH₂)ₖ- (wherein k, l, and m each represent an integer of 1 to 8 and R⁸ represents a hydrogen atom or a lower alkyl group));
X and Y may be the same or different and each represents -CH₂-, -NH-, or -O-; and n represents an integer of 0 to 4.

3. A chondrogenesis promoter according to claim 2, wherein the compound represented by general formula (I) is the compound represented by the following formula:

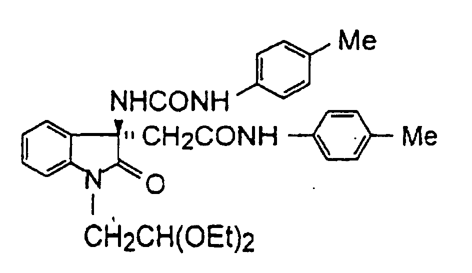

4. A cartilage repair agent comprising as an active ingredient a chondrogenesis promoter according to claim 1.

5. A chondrocyte phenotype maintaining agent comprising as an active ingredient a chondrogenesis promoter according to claim 1.

6. A bone fracture repair promoter comprising as an active ingredient a chondrogenesis promoter according to claim 1.

7. A therapeutic or prophylactic agent for a cartilage disease comprising as an active ingredient a chondrogenesis promoter according to claim 1.

8. A therapeutic or prophylactic agent according to claim 7, wherein the cartilage disease is a disease selected from the group consisting of osteoarthritis, chronic rheumatoid arthritis, osteochondritis dissecans, articular cartilage damage, herniated intervertebral disk, anotia, and microtia cartilage defect.

9. A reagent for biological, physical, or chemical research on cartilage, comprising a chondrogenesis promoter according to claim 1.

10. An agent for chondrocyte transplantation, comprising a chondrogenesis promoter according to claim 1.

11. A method for promoting chondrogenesis, comprising administering to a patient an effective amount of a chondrogenesis promoter according to claim 1.

12. A therapeutic or prophylactic method for a cartilage disease, comprising administering to a patient an effective amount of a chondrogenesis promoter according to claim 1.

13. A low molecular weight compound having a chondrogenesis promoting effect for use in combination with a compound with MEK1 inhibitory activity, wherein the low molecular weight compound comprises as an active ingredient a compound represented by general formula (I) or a salt thereof: 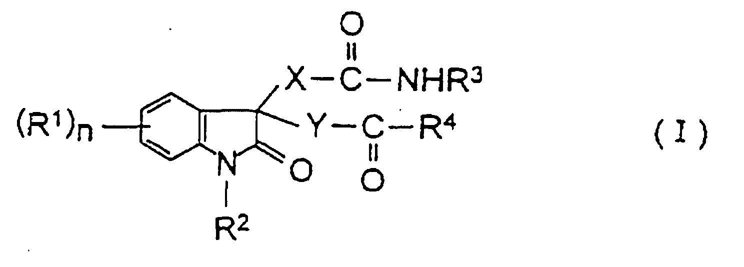 wherein
R¹ represents a halogen atom, a lower alkyl group, a lower alkoxy group, a hydroxyl group, a nitro group, a trifluoromethyl group, a lower alkylthio group, an acyl group, a carboxyl group, a mercapto group, or an amino group with an optional substituent;
R² represents a hydrogen atom, a lower alkyl group with an optional substituent, a lower alkenyl group with an optional substituent, a lower alkynyl group with an optional substituent, a lower alkoxy group with an optional substituent, an acyl group with an optional substituent, an aryl group with an optional substituent, or a heterocyclic group with an optional substituent;
R³ represents a lower alkyl group with an optional substituent, a cycloalkyl group with an optional substituent, an aryl group with an optional substituent, or a heterocyclic group with an optional substituent;
R⁴ represents a hydrogen atom, a lower alkyl group with an optional substituent, an aryl group with optional substituent, a heterocyclic group with an optional substituent, -OR⁵, -SR⁵ or -NR⁶R⁷ (wherein R⁵, R⁶ and R⁷ may be the same or different and each represents a hydrogen atom, a lower alkyl group with an optional substituent, a cycloalkyl group with an optional substituent, an aryl group with an optional substituent, a heterocyclic group with an optional substituent, a lower alkoxy group, or an amino group with an optional substituent, and R⁶ and R⁷ may together form a group represented by -(CH₂)ₘ- or -(CH₂)ₗNR⁸(CH₂)ₖ- (wherein k, l, and m each represent an integer of 1 to 8 and R⁸ represents a hydrogen atom or a lower alkyl group));
X and Y may be the same or different and each represents -CH₂-, -NH-, or -O-; and n represents an integer of 0 to 4.

14. A low molecular weight compound having a chondrogenesis promoting effect according to claim 13, wherein the compound represented by general formula (I) is a compound represented by the following formula:

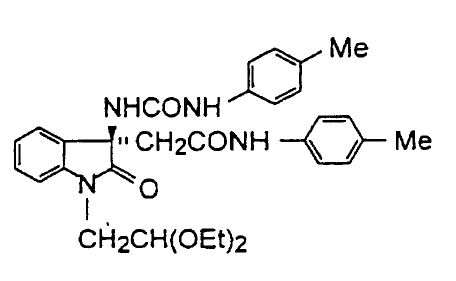

15. A therapeutic or prophylactic method for a cartilage disease, comprising administering to a patient a low molecular weight compound having a chondrogenesis promoting effect according to claim 13 and a compound with MEK1 inhibitory activity simultaneously, or within an interval in which the effect of combined use can substantially appear.

16. A therapeutic or prophylactic method for a cartilage disease, comprising administering to a patient, a low molecular weight compound having a chondrogenesis promoting effect according to claim 14 and a compound with MEK1 inhibitory activity simultaneously, or within an interval in which the effect of combined use can substantially appear.

17. A therapeutic or prophylactic method according to claim 12, 15, or 16, wherein the cartilage disease is a disease selected from the group consisting of osteoarthritis, chronic rheumatoid arthritis, osteochondritis dissecans, articular cartilage damage, herniated. intervertebral disk, anotia, and microtia cartilage defect.

18. A kit comprising a first container for containing the low molecular weight compound having a chondrogenesis promoting effect according to claim 13 or 14, and a second container for containing a compound with MEK1 inhibitory activity.

19. A kit according to claim 18, further comprising instructions for administering to a patient the low molecular weight compound having a chondrogenesis promoting effect and the compound with MEK1 inhibitory activity.

20. A method for proliferating chondrocytes comprising culturing chondrocytes in the presence of the chondrogenesis promoter according to claim 1 to obtain proliferated chondrocytes.

21. A method for proliferating chondrocytes according to claim 20, wherein extracellular matrices are resultantly formed along with proliferation of the chondrocyte to obtain proliferated chondrocytes containing the extracellular matrices.

22. A method for proliferating chondrocytes according to claim 20 wherein the proliferated chondrocytes are intended for chondrocyte transplantation.

23. A method for proliferating chondrocytes according to claim 22 wherein the chondrocyte transplantation is autologous chondrocyte transplantation.

24. A method for producing chondrocytes comprising culturing undifferentiated mesenchymal cells in the presence of the chondrogenesis promoter according to claim 1, wherein the chondrogenesis promoter promotes differentiation of the undifferentiated mesenchymal cells into the chondrocytes.

25. A method for producing chondrocyte according to claim 24, wherein extracellular matrices are resultantly formed along with proliferation of the chondrocyte to obtain proliferated chondrocytes containing the extracellular matrices.

26. A method for producing chondrocyte according to claim 24, wherein the chondrocytes obtained by promoting differentiation of undifferentiated mesenchymal cells into chondrocytes are intended for chondrocyte transplantation.

27. A method for producing chondrocyte according to claim 26 wherein the chondrocyte transplantation is autologous chondrocyte transplantation.

28. A chondrocyte obtained by the method for proliferating chondrocyte according to claim 20.

29. A chondrocyte obtained by the method for proliferating chondrocyte according to claim 21.

30. A chondrocyte obtained by the method for producing chondrocyte according to claim 24.

31. A chondrocyte obtained by the method for producing chondrocyte according to claim 25.

32. A chondrogenesis promoter according to claim 1 wherein the chondrogenesis promoter has growth properties of chondrocytes.

33. A chondrogenesis promoter according to claim 1 wherein the chondrogenesis promoter has properties of extracellular matrix formation.

34. A chondrogenesis promoter according to claim 1, wherein the chondrogenesis promoter has properties of differentiation of undifferentiated mesenchymal cells into chondrocytes.
